# EUROPEAN PATENT APPLICATION

(11) **EP 3 061 394 A1**
(43) Date of publication of application: **31.08.2016**
(21) Application number: 16156658.3
(22) Date of filing: 22.02.2016
(51) Int. Cl.: A61B 5/024, A61B 5/00, A61B 5/1455, A61B 5/1495, G01N 21/27, A61B 5/145

(54) **INFORMATION ACQUISITION APPARATUS**

(30) Priority: 24.02.2015 JP 2015033759
(71) Applicant: Seiko Epson Corporation, Tokyo 163 (JP)
(72) Inventor: Ishiguro, Hideto, Nagano, 392-8502 (JP); Eguchi, Tsukasa, Nagano, 392-8502 (JP); Tsuchiya, Hitoshi, Nagano, 392-8502 (JP); Ito, Megumu, Nagano, 392-8502 (JP)
(74) Representative: Hoffmann Eitle

(57) **Abstract**

An information acquisition apparatus (1, 115, 126) comprises: a first unit (2) including a photoreceiver (10) for receiving first reflected light reflected at an object (4), and outputting a signal corresponding to light intensity of the first reflected light; and a second unit (3) separately provided from the first unit (2), the second unit (3) including a calibrator (36) having a stable reflectance and for outputting second reflected light to the photoreceiver (10), the second reflected light being used for comparing the light intensity of the first reflected light.

## Description

### BACKGROUND

### 1. Technical Field

The present invention relates to an information acquisition apparatus.

### 2. Related Art

Apparatuses that acquire biological information of a subj ect in a noninvasive fashion are in use. Such apparatuses put a small burden on subjects, and have high safety. One such apparatus is disclosed in JPH11-323 A as a noninvasive blood analyzing apparatus that acquires information of blood components using light. According to this publication, a sensor is brought into contact with the subject's skin surface, and measurement light is applied into the body of the subject. Hemoglobin in the blood absorbs light of specific wavelengths. The reflected light from the subject is analyzed to detect the proportion of the oxygenated form of hemoglobin. The apparatus also detects biological information such as information of blood components.

The apparatus described in the foregoing publication detects blood information from a blood vessel selected as a test object. The apparatus emits light from a light source unit, and an imaging section receives light. The light source unit and the imaging section are electronic components, and undergo changes over time. The quantity of light from the light source decreases, and the imaging section lowers its sensitivity to light. The accuracy of the detected light information thus decreases with time. There accordingly is a need for an information acquisition apparatus that can accurately detect the characteristics of reflected light from an object even when sensor sensitivity changes with time.

### SUMMARY

An advantage of some aspects of the invention is to solve the problems described above, and the invention can be implemented as the following forms or application examples.

### Application Example 1

An information acquisition apparatus according to this application example includes: a first unit including a photoreceiver that receives first reflected light reflected at an object, and that outputs a signal corresponding to light intensity of the first reflected light; and a second unit separately provided from the first unit, the second unit including a calibrator that has a stable reflectance and that outputs second reflected light to the photoreceiver, the second reflected light being used for comparing the light intensity of the first reflected light.

According to this application example, the information acquisition apparatus includes the first unit and the second unit. The first unit and the second unit are separable from each other. Upon receiving the first reflected light, the photoreceiver outputs a signal corresponding to the light intensity of the first reflected light reflected at the object. Upon reflecting light, the object absorbs light of a specific wavelength that varies with the component of the object. Information of the object can thus be acquired by analyzing the output light intensity of the first reflected light from the photoreceiver.

The second unit includes the calibrator that outputs to the photoreceiver the second reflected light used to compare the light intensity of the first reflected light. Upon receiving the second reflected light, the photoreceiver outputs a signal corresponding to the light intensity of the reflected light at the calibrator. The light source of the light applied to the calibrator and the object undergoes changes with time, and the rate at which the photoreceiver converts the reflected light into a signal also varies with time. On the other hand, the reflectance of the calibrator remains stable over extended time periods. The amount of change of the detected light intensity of the reflected light at the calibrator has a correlation with changes occurring in the light applied to the calibrator and the object, and changes occurring in the rate at which the photoreceiver converts the reflected light into a signal. The amount of change of the detected light intensity of the reflected light at the calibrator, and the detected light intensity of the reflected light at the object can thus be used to accurately detect the characteristics of the reflected light at the object.

### Application Example 2

In the information acquisition apparatus according to the application example, the first unit and the second unit include locating sections with which the photoreceiver and the calibrator are installed face to face.

According to this application example, the first unit and the second unit have locating sections. The photoreceiver and the calibrator are installed face to face with the locating sections. This ensures that the photoreceiver receives the second reflected light from the calibrator.

### Application Example 3

In the information acquisition apparatus according to the application example, the photoreceiver includes: a light-emitting device that emits light applied to the calibrator or the object; and a light-receiving device that receives the second reflected light or the first reflected light, the light-emitting device and the light-receiving device having optical axes in the same direction.

According to this application example, the photoreceiver includes the light-emitting device and the light-receiving device. The light-emitting device and the light-receiving device have optical axes in the same direction. The light-emitting device emits light in a predetermined directional characteristic. The direction with the highest light quantity in the light of this directional characteristic is the optical axis of the light-emitting device. The light-receiving device has a predetermined directional characteristic for the sensitivity of the light it receives. The direction with the highest sensitivity in the sensitivity directional characteristic is the optical axis of the light-receiving device. In the photoreceiver, the direction with a high emission quantity and the direction with the highest photoreception sensitivity are the same.

The photoreceiver can thus receive the second reflected light with good sensitivity with the calibrator installed in the direction of the optical axes of the light-emitting device and the light-receiving device. Likewise, the photoreceiver can receive the first reflected light with good sensitivity with the object placed in the direction of the optical axes of the light-emitting device and the light-receiving device.

### Application Example 4

In the information acquisition apparatus according to the application example, the calibrator contains polytetrafluoroethylene.

According to this application example, the calibrator contains polytetrafluoroethylene. Polytetrafluoroethylene reflects near-infrared light without absorbing it. This makes it possible to efficiently obtain the second reflected light used for calibration.

### Application Example 5

In the information acquisition apparatus according to the application example, the first unit includes: a glucose level arithmetic section that computes a glucose level using an output signal from the photoreceiver of which signal corresponds to light intensity of the first reflected light; a determining section that compares the glucose level with a determination value to determine whether the object is in an abnormal state; and a warning section that gives a warning when the object is in an abnormal state.

According to this application example, the information acquisition apparatus includes the glucose level arithmetic section, the determining section, and the warning section. The glucose level arithmetic section computes a glucose level using an output signal from the photoreceiver of which signal corresponds to light intensity of the first reflected light. The determining section compares the glucose level with a determination value to determine whether the object is in an abnormal state. The warning section gives a warning when it is determined that the object is in an abnormal state. This makes it possible to immediately notify the obj ect of an abnormal state when the object is in an abnormal state.

### Application Example 6

In the information acquisition apparatus according to the application example, the first unit includes a sending section that sends information of the glucose level, and the second unit includes a receiving section that receives information of the glucose level, and a storage section that stores information of the glucose level.

According to this application example, the first unit includes the sending section, and the second unit includes the receiving section. The first unit sends glucose level information to the second unit. The second unit includes the storage section, and the glucose level information is stored in the storage section. The storage section can store long-term information of glucose levels. This makes it possible to analyze the trend of glucose level changes over extended time periods.

### Application Example 7

In the information acquisition apparatus according to the application example, the second unit includes an analysis arithmetic section that analyzes information of the glucose level.

According to this application example, the analysis arithmetic section analyzes information of glucose levels. The storage section of the second unit stores long-term information of glucose levels. The analysis arithmetic section can thus analyze long-term patterns of glucose levels, and long periodic changes of glucose levels.

### Application Example 8

In the information acquisition apparatus according to the application example, the analysis arithmetic section selects a countermeasure for the object, and the second unit includes a display section that displays the countermeasure.

According to this application example, the analysis arithmetic section selects a countermeasure for the object. The display section displays the countermeasure. This makes it possible to present to the object ways to maintain normal glucose levels.

### BRIEF DESCRIPTION OF THE DRAWINGS

The invention will be described with reference to the accompanying drawings, wherein like numbers reference like elements.
FIG. 1A is a schematic view explaining an installation example of a component measurement apparatus (information acquisition apparatus) according to First Embodiment, and FIGS. 1B and 1C are schematic plan views representing the structure of a first unit.
FIG. 2 is an exploded perspective view representing the structure of the first unit.
FIG. 3A is a schematic plan view representing the structure of a sensor module, FIG. 3B is a schematic side sectional view representing the structure of the sensor module, and FIG. 3C is a partial schematic side sectional view explaining an operation of the sensor module.
FIG. 4A is a schematic perspective view representing the structure of a second unit, FIG. 4B is a schematic plan view representing the contacting structure of the first unit and the second unit, and FIG. 4C is a schematic side view representing a structure in which the first unit and the second unit are in contact with each other.
FIG. 5 is a block diagram representing the electrical control of the first unit.
FIG. 6 is a block diagram representing the electrical control of the second unit.
FIG. 7 is a flowchart of an information acquisition method.
FIG. 8 is a flowchart representing a maintenance step (step S1) in detail.
FIG. 9 is a flowchart representing an object measurement step (step S3) in detail.
FIGS. 10A to 10D are schematic views explaining the biological information acquisition method.
FIGS. 11A to 11C are schematic views explaining the biological information acquisition method.
FIGS. 12A to 12D are schematic views explaining the biological information acquisition method.
FIGS. 13A to 13C are schematic views explaining the biological information acquisition method.
FIGS. 14A to 14D are schematic views explaining the biological information acquisition method.
FIG. 15A is a block diagram representing a relevant portion of a sensor drive circuit according to Second Embodiment, and FIG. 15B is a flowchart representing a maintenance step (step S1) in detail.
FIG. 16 is a flowchart representing an object measurement step (step S3) in detail.
FIG. 17A is a block diagram representing a relevant portion of a sensor drive circuit according to Third Embodiment, and FIG. 17B is a flowchart representing a maintenance step (step S1) in detail.

### DESCRIPTION OF EXEMPLARY EMBODIMENTS

Embodiments are described below with reference to the accompanying drawings.

Note that the members in the drawings are shown in sizes that make the members recognizable in the drawings, and are not to scale relative to actual size or each other.

### First Embodiment

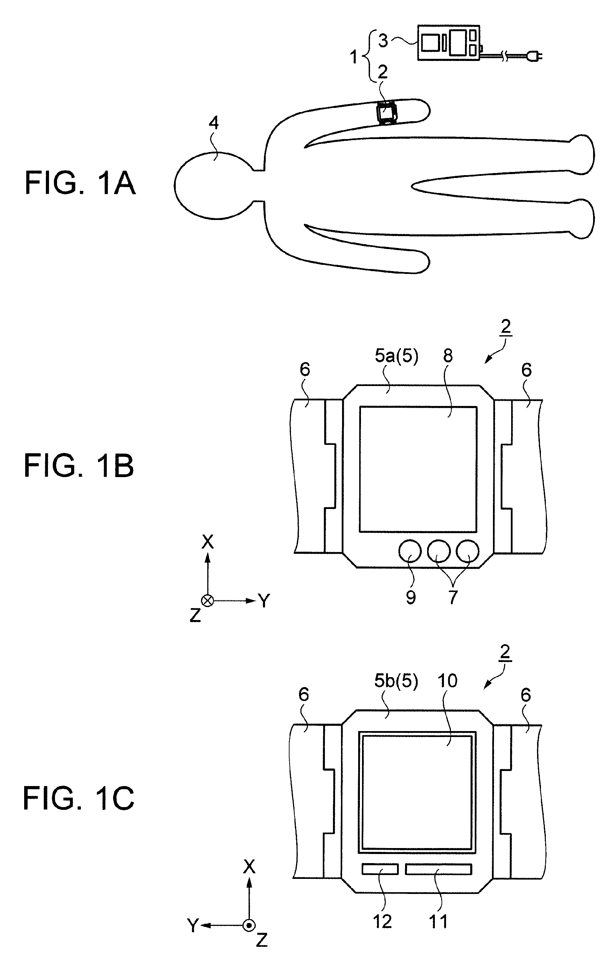

The present embodiment describes typical examples of a component measurement apparatus (information acquisition apparatus), and a component information acquisition method that analyzes blood components using the component measurement apparatus, with reference to the accompanying drawings. A component measurement apparatus according to First Embodiment is described with reference to FIGS. 1A to FIG. 6. FIG. 1A is a schematic view explaining an installation example of the component measurement apparatus. As illustrated in FIG. 1A, the component measurement apparatus 1 as an information acquisition apparatus is configured from a first unit 2 and a second unit 3. The first unit 2 is installed on a wrist of a subject 4 (object). The second unit 3 is separately installed from the first unit 2, and functions to assist the first unit 2. The component measurement apparatus 1 is a medical device for measuring blood components of the subject 4 in a noninvasive fashion, and represents medical equipment. The component measurement apparatus 1 measures components of the blood flowing in the blood vessels in the wrist. In the present embodiment, for example, the blood component measured is glucose concentration. Glucose concentration measurement enables measuring glucose levels.

FIGS. 1B and 1C are schematic plan views representing the structure of the first unit. FIG. 1B shows the top surface of the first unit 2. FIG. 1C shows the back surface of the first unit 2. As illustrated in FIG. 1B, the first unit 2 has a shape similar to the shape of a wrist watch. The first unit 2 has a first exterior portion 5. The first exterior portion 5 has a fixing band 6 on both sides (left and right in the figure) . The fixing band 6 is used to fix the component measurement apparatus 1 to a measured portion such as the wrist and arm of the subject 4. The fixing band 6 uses a Magic Tape^{®}. In referring to the component measurement apparatus 1, Y direction is the direction of extension of the fixing band 6, and X direction in the direction of extension of the arm of the subject 4. The direction in which the component measurement apparatus 1 faces the subject 4 is Z direction. X-, Y-, and Z-directions are orthogonal to each other.

The first exterior portion 5 has a surface 5a that faces outward upon mounting the first unit 2 on the subject 4. On the surface 5a of the first exterior portion 5 are installed operation switches 7, a touch panel 8, and a speaker 9 (warning section). The subject 4 enters measurement start instructions through the operation switches 7 and the touch panel 8. The touch panel 8 displays measurement result data. The component measurement apparatus 1 through the speaker 9 produces a warning sound to caution the subject 4.

As shown in FIG. 1C, a sensor module 10 is installed as a photoreceiver on the back surface 5b side of the first exterior portion 5. When in use, the sensor module 10 is brought close to the skin of the subject 4. The sensor module 10 applies measurement light to the skin of the subject 4, and receives reflected light. The sensor module 10 is a thin image sensor with a built-in light source and photosensor array. A communication connector 11 for communicating with external devices is installed on the back surface 5b of the first exterior portion 5. On the communication connector 11 are arranged contacts that contact and communicate with the second unit 3. A power connector 12 used to charge a rechargeable battery (not illustrated) is also installed. The power connector 12 is a connector that contacts the second unit 3 to receive power.

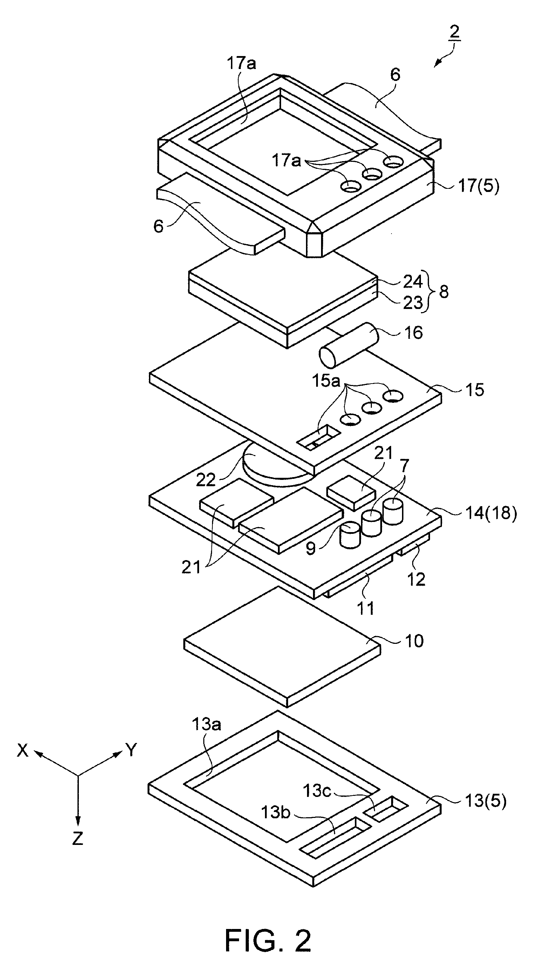

FIG. 2 is an exploded perspective view illustrating the structure of the first unit. As illustrated in FIG. 2, the first unit 2 is configured from a caseback 13, the sensor module 10, a circuit unit 14, a spacer 15, the touch panel 8, a vibrator (warning section) 16, and a top case 17, which are stacked in this order in Z direction. The caseback 13 and the top case 17 constitute the first exterior portion 5. The sensor module 10, the circuit unit 14, the spacer 15, the touch panel 8, and the vibrator 16 are housed in the first exterior portion 5.

The caseback 13 is a plate-shaped member that comes into contact with the subject 4. The caseback 13 has a quadrangular first window portion 13a installed on X direction side. The first window portion 13a is where the sensor module 10 is exposed. A light transmissive plate such as glass may be disposed in the first window portion 13a. This makes it possible to prevent entry of dust into the first exterior portion 5 through the first window portion 13a. Such a plate also can prevent contamination of the sensor module 10. The caseback 13 has a quadrangular second window portion 13b and third window portion 13c on - X direction side. The second window portion 13b is where the communication connector 11 is exposed. The third window portion 13c is where the power connector 12 is exposed.

The sensor module 10 is a sensor with light-emitting devices, light-receiving devices, and spectral devices installed in a grid. The sensor module 10 applies light to the subject 4, and detects the intensity of reflected light of specific wavelengths. The circuit unit 14 has a circuit board 18. On the circuit board 18 is installed an electrical circuit 21 that drives and controls the vibrator 16, the sensor module 10, and the touch panel 8. The electrical circuit 21 is configured from a plurality of semiconductor chips. The operation switches 7, the speaker 9, the communication connector 11, the power connector 12, and a rechargeable battery 22 are also installed on the circuit board 18. The rechargeable battery 22 is electrically connected to the power connector 12, and is chargeable via the power connector 12.

The spacer 15 is a structure installed between the circuit unit 14 and the touch panel 8. With the plurality of devices installed on the surface of the circuit unit 14 on - Z direction side, the circuit unit 14 has irregularities on this surface. The spacer 15 is installed over the circuit board 18, and provides a flat surface against the touch panel 8. The spacer 15 has a plurality of holes 15a, and the operation switches 7, the speaker 9, and the vibrator 16 penetrate through the holes 15a.

The touch panel 8 is structured to include a first display section 23, and an operation input section 24 installed on the first display section 23. The first display section 23 is not particularly limited, as long as it can display electronic data in the form of an image. The first display section 23 may be, for example, a liquid crystal display device, or an OLED (organic light-emitting diode) display device. In the present embodiment, the first display section 23 uses, for example, OLED.

The operation input section 24 is an input section with transparent electrodes disposed in a grid on a surface of a transparent plate. Upon an operator touching the transparent electrodes, current passes across the crossing electrodes, and enables detection of the location touched by the operator. The transparent plate may be a resin sheet or a glass plate, as long as it is light transmissive. The transparent electrodes may be, for example, IGO (indium-gallium oxide), ITO (indium Tin Oxide), or ICO (indium-cerium oxide), as long as it is a light-transmissive conductive film. The first display section 23 displays information such as a measurement status, and measurement results. The operation switches 7 are switches used to operate the component measurement apparatus 1, as is the operation input section 24. An operator operates the operation input section 24 and the operation switches 7 to enter various instructions, such as an instruction for starting a measurement of glucose level, and measurement conditions.

The vibrator 16 is installed on + Z direction side of the top case 17. The vibrator 16 is adapted to vibrate the first exterior portion 5. The component measurement apparatus 1 can function to caution the subject 4 with the vibration of the first exterior portion 5. The member used to constitute the vibrator 16 is not particularly limited, as long as it can vibrate the first exterior portion 5. In the present embodiment, for example, the vibrator 16 is a piezoelectric element.

The top case 17 has a plurality of holes 17a. The operation input section 24, the operation switches 7, and the speaker 9 are exposed through the holes 17a. The components from the sensor module 10 to the touch panel 8 are housed between the caseback 13 and the top case 17.

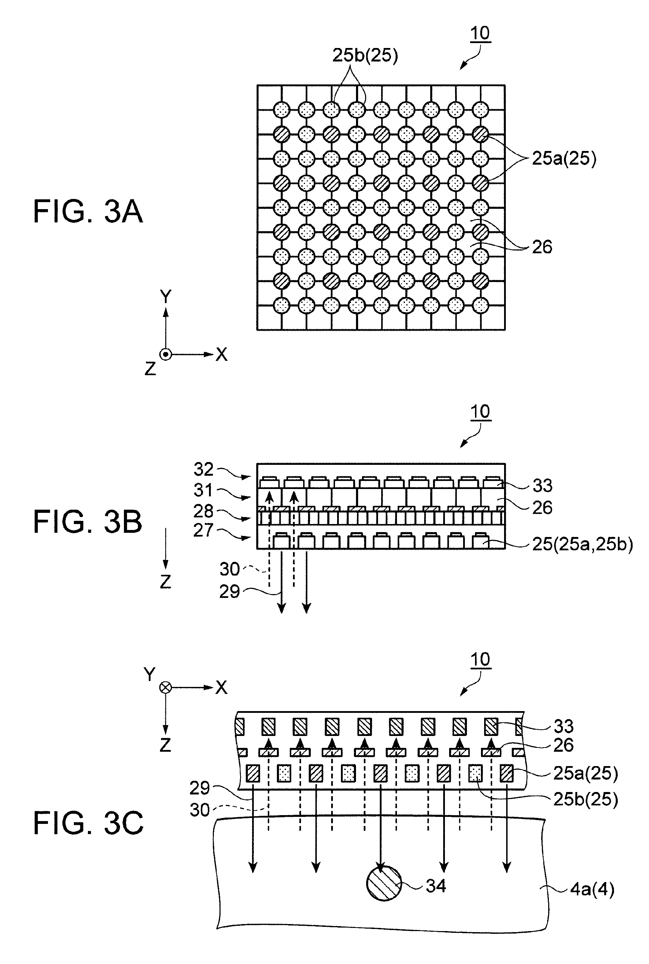

FIG. 3A is a schematic plan view showing the structure of the sensor module 10, as viewed from the back surface 5b side. FIG. 3B is a schematic side sectional view illustrating the structure of the sensor module. FIG. 3C is a partial schematic side sectional view explaining the operation of the sensor module. As illustrated in FIG. 3A, the sensor module 10 has a two-dimensional array of light-emitting devices 25 in a grid. Between the adjacent light-emitting devices 25 are installed spectral devices 26.

The arrayed directions of the light-emitting devices 25 and the spectral devices 26 are X and Y directions. The light-emitting devices 25 and the spectral devices 26 are disposed at the same intervals in X and Y directions. The light-emitting devices 25 and the spectral devices 26 are disposed in a staggered fashion in X and Y directions with a predetermined distance in between. Accordingly, the spectral devices 26 have wide non-overlapping portions with the light-emitting devices 25 as viewed from the back surface 5b side. This structure permits light propagating from the subject 4 side to reach the spectral devices 26.

The light-emitting devices 25 are configured from imaging light-emitting devices 25a and measurement light-emitting devices 25b. In the figure, the rows are in + Y direction from the bottom. The 1st, 3rd, 5th, 7th, and 9th rows are configured from the measurement light-emitting devices 25b. The imaging light-emitting devices 25a and the measurement light-emitting devices 25b are alternately disposed in the 2nd, 4th, 6th, and 8th rows in the figure. The light-emitting devices 25 are installed in such a manner that four light-emitting devices 25 surround a single spectral device 26. A unit of four light-emitting devices 25 includes one imaging light-emitting device 25a, and three measurement light-emitting devices 25b.

In capturing an image to detect locations of blood vessels, the imaging light-emitting devices 25a apply light to the subject 4. The light applied by the imaging light-emitting devices 25a has a 700 nm to 900 nm wavelength range centered at 800 nm. Hemoglobin in the blood has high absorption of light at 800 nm wavelength. An image of blood vessel locations can thus be captured with light applied to the subject 4 by the imaging light-emitting devices 25a.

In detecting blood glucose concentration, the measurement light-emitting devices 25b apply light to the subject 4. The light applied by the measurement light-emitting devices 25b has a 900 nm to 2000 nm wavelength range centered at 1450 nm. Glucose in the blood has high absorption of light at 1200 nm, 1600 nm, and 2000 nm wavelengths. Blood glucose concentration can thus be detected with light applied to the subject 4 by the measurement light-emitting devices 25b. Glucose is also called grape sugar.

For simplicity, the light-emitting devices 25 are shown as an array of 9 rows and 9 columns. The number of rows and the number of columns in the array of the light-emitting devices 25 and the spectral devices 26 are not particularly limited, and may be appropriately set. For example, the interval between these devices is preferably 1 to 1500 µm. Considering the balance between manufacturing cost and measurement accuracy, the interval is more preferably, for example, about 100 to 1500 µm. The light-emitting devices 25 and the spectral devices 26 are not limited to the layered configuration, and these may be disposed side by side on a plane. In the present embodiment, for example, 250 rows x 250 columns of light-emitting devices 25 are installed. The interval between the light-emitting devices 25 is not particularly limited either. In the present embodiment, for example, the interval between the light-emitting devices 25 is 0.1 mm. The sensor module 10 can thus also function as an imaging device.

As illustrated in FIG. 3B, the array of light-emitting devices 25 constitutes a light-emitting layer 27 (light source). The light-emitting devices 25 represent an irradiator that applies measurement light. The light-emitting devices 25 are not particularly limited, as long as it can emit near-infrared rays that can pass through the subcutaneous tissue. The light-emitting devices 25 may use, for example, LED (light emitting diode), or OLED (organic light-emitting diode).

A light-shielding layer 28 is installed over the light-emitting layer 27. The measurement light 29 emitted by the light-emitting layer 27 toward the subject 4 is reflected at the subcutaneous tissue of the subject 4, and becomes reflected light 30. The light-shielding layer 28 passes light directed to the spectral devices 26, but selectively blocks other light. A spectral layer 31 is installed over the light-shielding layer 28. The spectral devices 26 are arrayed in a grid in the spectral layer 31. The spectral devices 26, also called etalons, are devices that selectively pass near-infrared rays of predetermined wavelengths. The spectral devices 26 in response to an input instruction signal pass reflected light 30 of the wavelength specified by the instruction signal. The spectral devices 26 include a pair of oppositely disposed mirrors, and an electrostatic actuator is installed that adjusts the distance between the mirrors. The passage of reflected light 30 of predetermined wavelengths is permitted by the electrostatic actuator adjusting the distance between the mirrors.

Glucose has peak wavelengths of 1200 nm, 1600 nm, and 2000 nm. Blood sugar level can be measured by detecting transmittance at these three wavelengths. The wavelengths of the reflected light 30 passed by the spectral devices 26 are not particularly limited. In the present embodiment, the spectral devices 26, for glucose detection, pass light of, for example, 1500 nm to 1700 nm wavelengths centered at 1600 nm.

A light-receiving layer 32 is installed over the spectral layer 31. The light-receiving layer 32 has a grid-like two-dimensional array of light-receiving devices 33. The light-receiving devices 33 are arrayed in the same pattern as the spectral devices 26. The light-receiving devices 33 overlie the spectral devices 26 as viewed in the direction of travel of the reflected light 30.

The light-receiving devices 33 output electrical signals according to the quantity of the reflected light 30 they receive. The light-receiving devices 33 may use, for example, imaging devices such as CCD (Charge Coupled Device Image Sensor), and CMOS (Complementary Metal Oxide Semiconductor Image Sensor), as long as light intensity can be converted into electrical signals. The light-receiving devices 33 each may have a configuration that includes a plurality of devices for receiving wavelength components necessary for calibration. The sensor module 10 has its front surface on the side of the light-emitting layer 27, and is installed on the back surface 5b of the first exterior portion 5 in such an orientation that the front surface side faces the skin surface of the subject 4.

The light-emitting devices 25 and the light-receiving devices 33 have optical axes in the same direction. The light-emitting devices 25 emit the measurement light 29 in a predetermined directional characteristic. The direction with the highest light quantity in the directional characteristic of the measurement light 29 represents the optical axis of the light-emitting device. The detection sensitivity of the light-receiving devices 33 for the reflected light 30 has a predetermined directional characteristic. The direction with the highest sensitivity in the sensitivity directional characteristic represents the optical axis of the light-receiving devices 33. In the sensor module 10, the direction with a high emission quantity and the direction with the highest photoreception sensitivity are the same. Specifically, the measurement light 29 and the light-receiving devices 33 have optical axes in the same direction. Accordingly, the sensor module 10 can receive the reflected light 30 with good sensitivity with a measured portion 4a placed on the optical axes of the light-emitting devices 25 and the light-receiving devices 33.

As illustrated in FIG. 3C, all the imaging light-emitting devices 25a in the sensor module 10 simultaneously emit light in capturing the location of a blood vessel 34. The location opposite the sensor module 10 represents the measured portion 4a. The measurement light 29 is applied over the whole region of the measured portion 4a of the subject 4. The measurement light 29 is reflected at the measured portion 4a, and becomes the reflected light 30. The reflected light 30 is received by all the light-receiving devices 33, and a biological image is acquired. For the measurement of blood components, only the specified devices in the measurement light-emitting devices 25b emit light, and the reflected light 30 is received by the specified devices in the light-receiving devices 33.

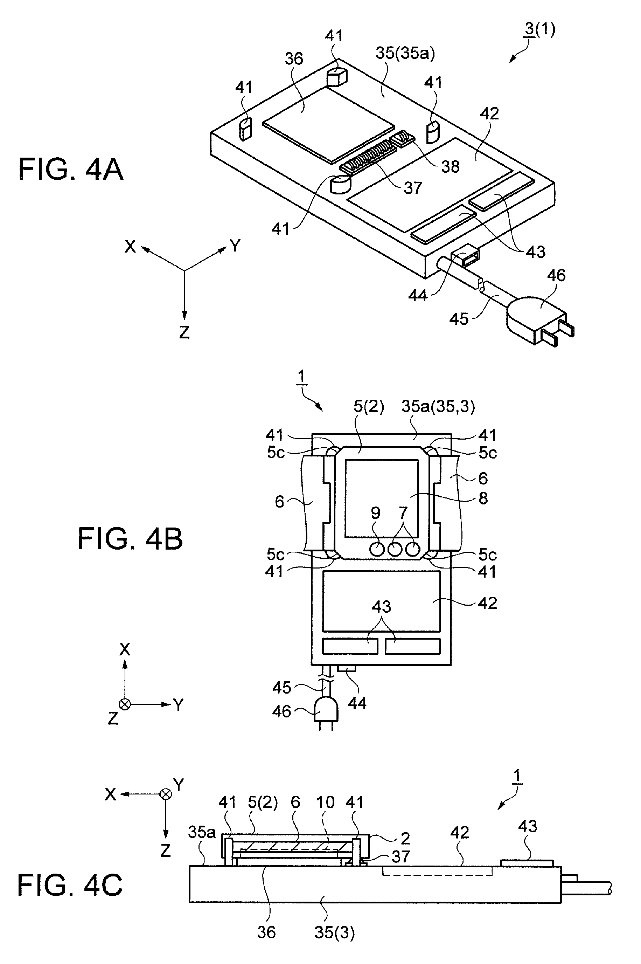

FIG. 4A is a schematic perspective view representing the structure of the second unit. FIG. 4B is a schematic plan view representing the contacting structure of the first unit and the second unit. FIG. 4C is a schematic side view representing a structure in which the first unit and the second unit are in contact with each other. As illustrated in FIG. 4A, the second unit 3 has a plate-shaped second exterior portion 35. The second exterior portion 35 has a thickness direction along Z direction. The second exterior portion 35 is rectangular in outer shape when viewed in Z direction. The longitudinal direction of the second exterior portion 35 as viewed in Z direction is along X direction, and the direction orthogonal to this longitudinal direction is Y direction.

The second exterior portion 35 has a surface 35a on - Z direction side. The surface 35a is a surface that contacts the first unit 2. A calibration plate 36 is installed as a calibrator on X direction side of the surface 35a. The calibration plate 36 has a shape of a quadrangular plate, and the sides of the quadrangle are longer than the sides of the sensor module 10. The material of the calibration plate 36 is not particularly limited, as long as it can stably reflect infrared light over extended time periods. Materials such as polytetrafluoroethylene, and metals may be used. Polytetrafluoroethylene is also called Teflon^{®}. In the present embodiment, for example, the calibration plate 36 is a plate produced by compacting and sintering polytetrafluoroethylene particles. The plate has a high reflectance of about 98% or more in a 1500 nm to 1700 nm wavelength region. The calibration plate 36 can thus efficiently output the reflected light 30 to the light-emitting devices 25.

A communication socket 37 and a power socket 38 are installed on - X direction side of the calibration plate 36. The communication socket 37 is a connection for the communication connector 11, and the first unit 2 and the second unit 3 communicate with each other via the communication connector 11 and the communication socket 37. The power socket 38 is a connection for the power connector 12, and the second unit 3 supplies power to the first unit 2 via the power connector 12 and the power socket 38.

Four locating projections 41 are installed as locating sections around the calibration plate 36, the communication socket 37, and the power socket 38. The first unit 2 is installed in contact with the second unit 3 in such a manner that the exterior of the first unit 2 contacts the locating projections 41. In this way, the locating projections 41 locate the first unit 2. A second display section 42 is installed on - X direction side of the communication socket 37 and the power socket 38. The second display section 42 is where the results of computations performed by the second unit 3, or text guidance to an operator are displayed. Operation switches 43 are installed on - X direction side of the second display section 42. Operation switches 43 are switches an operator presses to operate the second unit 3. An operator checking the second display section 42 can send instructions to the second unit 3 by operating the operation switches 43.

A communication outlet 44 and a power cable 45 are installed on the side surface of the second exterior portion 35 on - X direction side. The communication outlet 44 is a connection used to install a communication cable when communicating with external devices. The power cable 45 is an external power input cable, and has a power plug 46 at the end.

As illustrated in FIG. 4B, the first unit 2 is installed on the surface 35a of the second unit 3. Here, the first exterior portion 5 is installed along the locating projections 41. The first exterior portion 5 has locating receptacles 5c as locating sections that contact the locating projections 41. The locating receptacles 5c are shaped to fit the locating projections 41. This makes it possible to install the first unit 2 and the second unit 3 relative to each other with good repeatability.

As illustrated in FIG. 4C, the sensor module 10 and the calibration plate 36 are disposed face to face with the locating projections 41. This ensures that the light-receiving devices 33 receive the reflected light 30 in response to the light-emitting devices 25 outputting the measurement light 29.

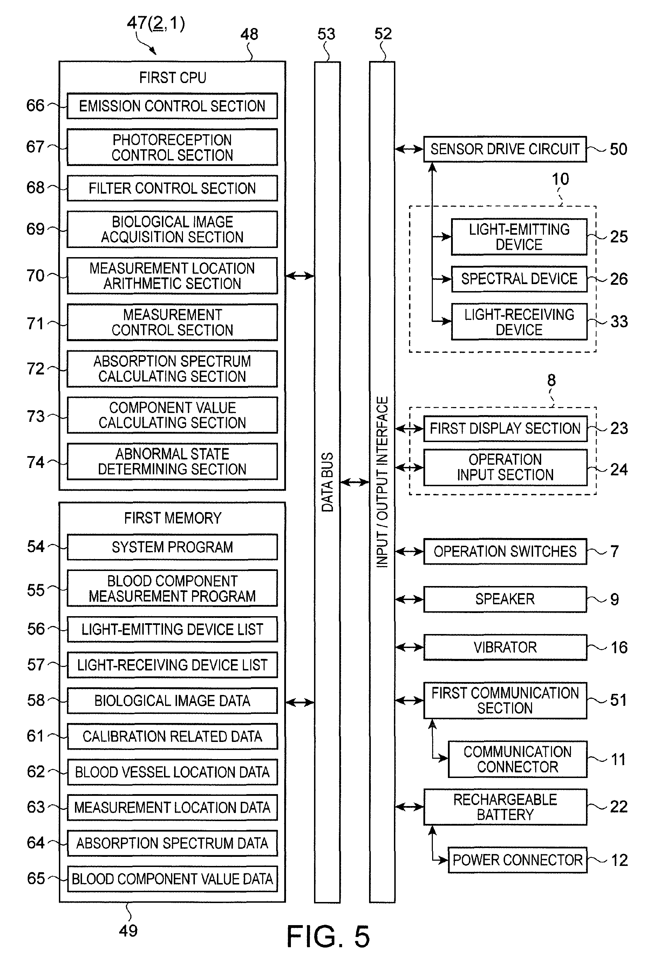

FIG. 5 is a block diagram representing the electrical control of the first unit. Referring to FIG. 5, the first unit 2 includes a first controller 47 that controls the operation of the first unit 2. The first controller 47 includes a first CPU 48 (Central Processing Unit) as a processor that performs various arithmetic processes, and first memory 49 that stores a variety of information. A sensor drive circuit 50, the operation input section 24, the first display section 23, the operation switches 7, the speaker 9, the vibrator 16, the first communication section 51 (sending section), and the rechargeable battery 22 are connected to the first CPU 48 via an input/output interface 52 and a data bus 53.

The sensor drive circuit 50 is a circuit that drives the sensor module 10. The sensor drive circuit 50 drives the light-emitting devices 25, the spectral devices 26, and the light-receiving devices 33 constituting the sensor module 10. The light-emitting devices 25, the spectral devices 26, and the light-receiving devices 33 are two-dimensionally arrayed in a grid in the sensor module 10. The sensor drive circuit 50 turns on and off the light-emitting devices 25 according to instruction signals from the first CPU 48. The sensor drive circuit 50 sets a wavelength for passage of reflected light 30 through the spectral devices 26, using an instruction signal from the first CPU 48. The sensor drive circuit 50 amplifies the light intensity signal of the light received by the light-receiving devices 33, and sends the signal to the first CPU 48 after converting it into a digital signal.

The first display section 23 displays predetermined information according to instructions from the first CPU 48. An operator operates the operation input section 24 according to the displayed content, and enters instruction content. The instruction content is sent to the first CPU 48.

The speaker 9 is an audio output unit, and makes various audio outputs according to instructions from the first CPU 48. The speaker 9 outputs notification sounds indicative of information such as the start and the end of a glucose level measurement, and occurrence of an error.

The vibrator 16 is a device that vibrates the first exterior portion 5. Because the first exterior portion 5 is in contact with the subject 4, the first unit 2 can caution the subject 4 by vibrating the first exterior portion 5. The subject 4 can be cautioned using the vibrator 16 when the use environment of the component measurement apparatus 1 does not permit making sound from the speaker 9.

The first communication section 51 is configured from circuits such as a wired communication circuit, and a communication control circuit. The communication connector 11 performs communications with the second unit 3. The first communication section 51 may be used as a wireless communication circuit to perform wireless communications with the second unit 3.

The rechargeable battery 22 supplies power for driving the first unit 2. The rechargeable battery 22 outputs data indicative of a charge level to the first CPU 48. The first CPU 48 is adapted to detect the power charged in the rechargeable battery 22. The rechargeable battery 22 is connected to the power connector 12, and charged by the second unit 3.

The first memory 49 is a concept that includes semiconductor memories such as RAM and ROM, and external memory devices such as a hard disc, and a DVD-ROM. Functionally, the first memory 49 has a storage region set therein to store a system program 54 that describes control procedures for the operation of the component measurement apparatus 1, and a storage region set therein to store a blood component measurement program 55 that describes arithmetic procedures for estimating blood components. The first memory 49 also has a storage region set therein to store a light-emitting device list 56 that represents data indicative of the locations of the light-emitting devices 25.

The first memory 49 also has a storage region set therein to store a light-receiving device list 57 that represents data indicative of the locations of the light-receiving devices 33. The first memory 49 also has a storage region set therein to store biological image data 58 obtained by capturing the location of the blood vessel 34 under the light emitted by all the light-emitting devices 25. The first memory 49 also has a storage region set therein to store calibration related data 61 used to calibrate light intensity. The first memory 49 also has a storage region set therein to store blood vessel location data 62 indicative of the location of the blood vessel 34 computed from the biological image data 58. The first memory 49 also has a storage region set therein to store measurement location data 63 indicative of the location of the blood vessel 34 being measured.

The first memory 49 also has a storage region set therein to store absorption spectrum data 64 that represents the optical transmittance of the measured blood. The first memory 49 also has a storage region set therein to store blood component value data 65 indicative of the blood concentrations of the measured blood components. The first memory 49 also has various other storage regions set therein to serve different purposes, including a storage region that serves as a work area for the first CPU 48, and a storage region that serves as temporary files.

The first CPU 48 controls the measurement of blood glucose concentration according to the system program 54 and the blood component measurement program 55 stored in the first memory 49. Specifically, the first CPU 48 has an emission control section 66 to realize its functions. The emission control section 66 controls the switching that selectively turns on and off the light-emitting devices 25. The first CPU 48 also has a photoreception control section 67. The photoreception control section 67 controls the acquisition of digital data of the light quantity received by the light-receiving devices 33. The first CPU 48 also has a filter control section 68. The filter control section 68 controls the sensor drive circuit 50 to switch the wavelength that can pass through the spectral devices 26.

The first CPU 48 also has a biological image acquisition section 69. The biological image acquisition section 69 acquires a biological image of a portion of body directly below the sensor module 10. The acquisition of a biological image is made possible by the appropriate use of biological image capturing techniques, such as a known vein authentication technique. Specifically, all the light-receiving devices 33 are used to capture an image under the light emitted by all the imaging light-emitting devices 25a of the sensor module 10. The captured image generates a biological image. The biological image acquired by the biological image acquisition section 69 is stored as the biological image data 58 in the first memory 49.

The first CPU 48 also has a measurement location arithmetic section 70. The measurement location arithmetic section 70 performs a predetermined image process on the biological image, and acquires blood vessel location data. Specifically, a vein pattern is identified from the biological image using a known image processing technique. For example, the biological image is subjected to pixel-wise binarization or filtering relative to a reference luminance. In the processed biological image, pixels with luminance values below the reference luminance indicate blood vessels, and pixels with luminance values equal to or greater than the reference luminance indicate a non-blood vessel region. The blood vessel location data acquired by the measurement location arithmetic section 70 is stored as blood vessel location data 62 in the first memory 49.

The measurement location arithmetic section 70 selects a measurement target by selecting a location of blood vessel 34 satisfying predetermined selection conditions. The location selected as the measurement target may be a single blood vessel 34, or more than one blood vessel 34. The data of the blood vessel 34 at the selected measurement target location is stored as the measurement location data 63 in the first memory 49.

The measurement location arithmetic section 70 selects a measurement light-emitting device 25b and a light-receiving device 33 that are to be driven for the blood vessel 34 at each measurement location. Specifically, the measurement location arithmetic section 70 selects a light-emitting device 25 and a light-receiving device 33 that lie on a straight line orthogonal to the center line of the blood vessel 34 at the measurement location. Here, the measurement light-emitting device 25b and the light-receiving device 33 are selected in such a manner that the distance between the measurement location and the light-emitting device 25, and the distance between the measurement location and the light-receiving device 33 take values close to the optimum distance. The measurement light-emitting device 25b so selected is stored as the light-emitting device list 56 in the first memory 49. The light-receiving device 33 so selected is stored as the light-receiving device list 57 in the first memory 49.

The first CPU 48 also has a measurement control section 71. The measurement control section 71 makes the sensor drive circuit 50 turn on the measurement light-emitting device 25b. The measurement control section 71 causes the sensor drive circuit 50 to drive the light-receiving device 33 for detection of the light intensity of the reflected light 30. Here, the light intensity is the light intensity of the light that has passed through the blood vessel 34.

The first CPU 48 also has an absorption spectrum calculating section 72. The absorption spectrum calculating section 72 generates an absorption spectrum of the measured blood vessel 34. Specifically, the absorption spectrum calculating section 72 calculates the transmittance T of the blood vessel 34 using the light intensity of the light received by the light-receiving device 33, and generates an absorption spectrum. The absorption spectrum so calculated is stored as the absorption spectrum data 64 in the first memory 49. The measurement may be made at one or more wavelengths λ. The wavelength λ varies with the measured blood component.

The first CPU 48 also has a component value calculating section 73 as a glucose level arithmetic section. The component value calculating section 73 calculates a glucose concentration using the absorption spectrum. The calculation of absorption spectrum may use analysis techniques such as multiple linear regression analysis, main component regression analysis, PLS regression analysis, and independent component analysis. When there is more than one blood vessel 34 at the measurement location, a glucose concentration is calculated from the average absorption spectrum of different blood vessels 34. The calculated value is stored as the blood component value data 65 in the first memory 49.

The first CPU 48 also has an abnormal state determining section 74 as a determining section. The abnormal state determining section 74 compares the glucose concentration calculated by the component value calculating section 73 with a determination value to make a determination. When there is abnormality in the glucose concentration, the abnormal state determining section 74 warns the subject 4 using the first display section 23, the speaker 9, and the vibrator 16.

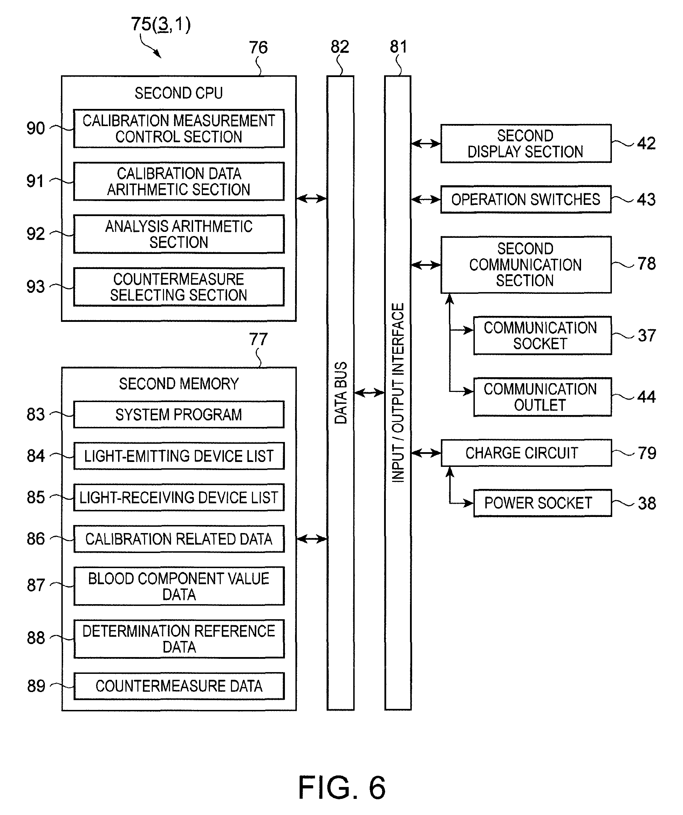

FIG. 6 is a block diagram representing the electrical control of the second unit. Referring to FIG. 6, the second unit 3 includes a second controller 75 that controls the operation of the second unit 3. The second controller 75 includes a second CPU 76 as a processor that performs various arithmetic processes, and second memory 77 that stores a variety of information. The second display section 42, the operation switches 43, a second communication section 78 (receiving section), and a charge circuit 79 are connected to the second CPU 76 via an input/output interface 81, and a data bus 82.

The second display section 42 displays predetermined information according to instructions from the second CPU 76. An operator operates the operation switches 43 according to the displayed content, and enters instruction content. The instruction content is sent to the second CPU 76.

The second communication section 78 is configured from circuits such as a wired communication circuit, and a communication control circuit. The second communication section 78 communicates with the first unit 2 via the communication socket 37. When the communication outlet 44 is connected to an external device (not illustrated), the second communication section 78 communicates with the external device via the communication outlet 44. The second communication section 78 may be used as a wireless communication circuit to perform wireless communications with the first unit 2 and external devices.

The charge circuit 79 is connected to the power socket 38, and charges the rechargeable battery 22 of the first unit 2 via the power socket 38. The charge circuit 79 can detect the start and the end of charging by detecting the current passing the power socket 38. The charge circuit 79 outputs to the second CPU 76 information concerning whether charging is in progress.

The second memory 77 is a concept that includes semiconductor memories such as RAM and ROM, and external memory devices such as a hard disc, and a DVD-ROM. Functionally, the second memory 77 has a storage region set therein to store a system program 83 that describes control procedures for the operation of the second unit 3 , and a storage region set therein to store a light-emitting device list 84 that represents data indicative of the locations of the light-emitting devices 25.

The second memory 77 also has a storage region set therein to store a light-receiving device list 85 that represents data indicative of the locations of the light-receiving devices 33. The second memory 77 also has a storage region set therein to store calibration related data 86 used to calibrate light intensity with a calibration plate 36. The second memory 77 also has a storage region set therein to store blood component value data 87 as glucose level information indicative of the blood concentration of the measured blood component. The second memory 77 also has a storage region set therein to store determination reference data 88 as reference data used to determine the blood component value data 87. The second memory 77 also has a storage region set therein to store countermeasure data 89 indicative of how to deal with abnormality when the result of the determination of the blood component value data 87 is abnormal. The second memory 77 also has various other storage regions set therein to serve different purposes, including a storage region that serves as a work area for the second CPU 76, and a storage region that serves as temporary files.

The second CPU 76 computes data used for calibration, or performs an arithmetic analysis of changes in blood glucose concentration, according to the system program 83 stored in the second memory 77. Specifically, the second CPU 76 has a calibration measurement control section 90 to realize this function. The calibration measurement control section 90 cooperates with the emission control section 66, the photoreception control section 67, and the filter control section 68 of the first CPU 48 to control the reflectance measurement of the calibration plate 36. The second CPU 76 also has a calibration data arithmetic section 91. The calibration data arithmetic section 91 uses the measured reflectance of the calibration plate 36 to check the performance of the light-emitting layer 27 and the light-receiving devices 33, and compute data for calibrating the output of the light-emitting layer 27 and the light-receiving devices 33. The second CPU 76 also has an analysis arithmetic section 92. The analysis arithmetic section 92 computes patterns in which the blood component value data 87 are changing. The second CPU 76 also has a countermeasure selecting section 93. When the changing patterns of the blood component value data 87 are not desirable for the subject 4, a countermeasure that is suited for the subject 4 is selected by the countermeasure selecting section 93 from the countermeasures stored in the countermeasure data 89, and the countermeasure selecting section 93 displays the selected countermeasure in the second display section 42.

The present embodiment has been described through the case where the functions of the first unit 2 are achieved by program software using the first CPU 48. However, these functions may be achieved with the use of an electronic circuit, when an electronic circuit (hardware) alone is sufficient to achieve the foregoing functions without using the first CPU 48. Similarly, the functions of the second unit 3, achieved by program software using the second CPU 76 in the foregoing embodiment, may be achieved with the use of an electronic circuit when an electronic circuit (hardware) alone is sufficient to achieve the foregoing functions without using the second CPU 76.

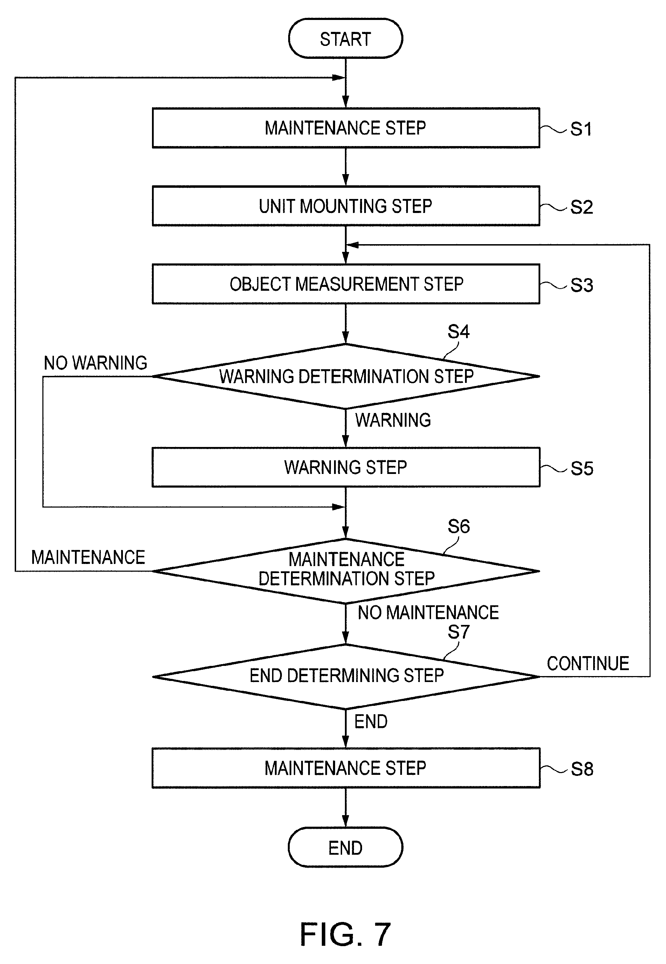

The following describes an information acquisition method that uses the component measurement apparatus 1 described above, with reference to FIG. 7 to FIG. 14D. FIG. 7 is a flowchart representing the information acquisition method. In the flowchart of FIG. 7, step S1 corresponds to a maintenance step, in which the charge circuit 79 charges the rechargeable battery 22 of the first unit 2. In step S1, the light-emitting devices 25 apply the measurement light 29 to the calibration plate 36, and the light-receiving devices 33 detect the reflected light 30. Step S1 is also a step in which the calibration data arithmetic section 91 calculates a calibration coefficient. The sequence then goes to step S2. Step S2 corresponds to a unit mounting step. In this step, an operator installs the first unit 2 on the subject 4. Step S3 is an obj ect measurement step. In this step, the measurement light 29 is applied to the measured portion 4a, and the light-receiving devices 33 detect the reflected light 30. Blood glucose is measured in this step. The sequence then goes to step S4. Step S4 is a warning determination step in which the abnormal state determining section 74 determines whether to warn the subject 4. When warning the subject 4 , the sequence goes to step S5 . The sequence goes to step S6 when not warning the subject 4.

Step S5 is a warning step. This step warns the subject 4 that an abnormal event has occurred. The sequence then goes to step S6. Step S6 is a maintenance determination step of determining whether to perform maintenance. When maintenance is performed, the sequence goes to step S1. The sequence goes to step S7 when not performing maintenance. Step S7 is an end determining step, which determines whether to continue or end the measurement. When continuing the measurement, the sequence goes to step S3. The sequence goes to step S8 when ending the measurement. Step S8 is a maintenance step. Step S8 is the same as step S1. This completes the information acquisition process.

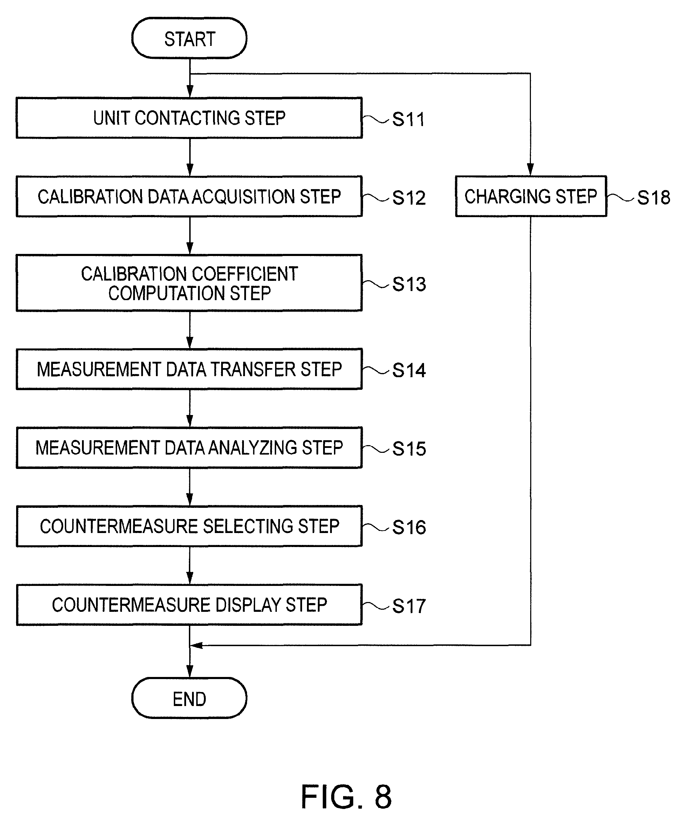

FIG. 8 is a flowchart representing the maintenance step (step S1) in detail. In the flowchart of FIG. 8, steps S11 to S17 and step S18 are performed in parallel. Step S11 corresponds to a unit contacting step. In this step, the first unit 2 is brought into contact with the second unit 3 by installing the first unit 2 on the second unit 3. The sequence then goes to step S12. Step S12 is a calibration data acquisition step. In this step, the measurement light 29 is applied to the calibration plate 36, and the reflected light 30 from the calibration plate 36 is detected. The sequence then goes to step S13.

Step S13 is a calibration coefficient computation step. In this step, the light intensity of the reflected light 30 is used to compute the calibration coefficient used in the object measurement step (step S3). Step S13 is also a step in which calibration coefficient data is transferred from the second unit 3 to the first unit 2. The sequence then goes to step S14. Step S14 is a measurement data transfer step. In this step, the blood component value data 65 is transferred from the first memory 49 of the first unit 2 to the second memory 77 of the second unit 3.

Step S15 is a measurement data analyzing step. In this step, the analysis arithmetic section 92 analyzes the blood component value data 65 to analyze the patterns in which the blood glucose concentration is changing. The sequence then goes to step S16. Step S16 is a countermeasure selecting step. In this step, a countermeasure to be performed by the subject 4 is selected from the countermeasure data 89 when the blood glucose concentration is showing an increasing pattern. The sequence then goes to step S17. Step S17 is a countermeasure display step. In this step, the countermeasure selected in step S16 is displayed. Step S18 is a charging step. In this step, the second unit 3 sends power to the first unit 2, and the rechargeable battery 22 is charged. This completes the maintenance step (step S1).

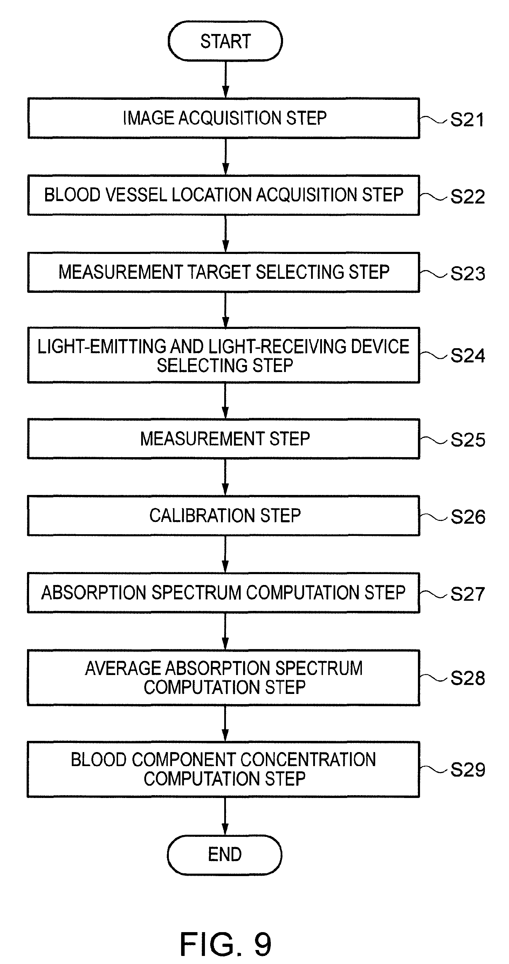

FIG. 9 is a flowchart representing the object measurement step (step S3) in detail. In the flowchart of FIG. 9, step S21 corresponds to an image acquisition step. In this step, the biological image acquisition section 69 simultaneously turns on all the imaging light-emitting devices 25a, and the light-receiving devices 33 of the light-receiving layer 32 capture an image of the blood vessel 34. The sequence then goes to step S22. Step S22 is a blood vessel location acquisition step. In this step, the image captured by the measurement location arithmetic section 70 is used to acquire the location of the blood vessel 34. The sequence then goes to step S23.

Step S23 is a measurement target selecting step. In this step, a location suited for measurement is selected from the blood vessel 34 in the measurement portion 4a by the measurement location arithmetic section 70. The measurement location arithmetic section 70 also selects a reference measurement location. The sequence then goes to step S24. Step S24 is a light-emitting and light-receiving device selecting step. In this step, the measurement location arithmetic section 70 selects a measurement light-emitting device 25b and a light-receiving device 33 that are to be driven for the measurement. The measurement location arithmetic section 70 also selects a measurement light-emitting device 25b and a light-receiving device 33 that are to be driven for the acquisition of reference data. The sequence then goes to step S25.

Step S25 is a measurement step. In this step, the measurement light-emitting device 25b applies the measurement light 29 to the measured portion 4a, and the light intensity of the reflected light 30 received by the light-receiving device 33 is measured. The sequence then goes to step S26. Step S26 is a calibration step. In this step, the light intensity measured by the calibration data arithmetic section 91 is multiplied by the calibration coefficient. The sequence then goes to step S27. Step S27 is an absorption spectrum computation step. In this step, the absorption spectrum calculating section 72 computes the blood transmittance using the measurement result data. The sequence then goes to step S28. Step S28 is an average absorption spectrum computation step, in which the blood transmittances at different measurement locations are used to compute the mean transmittance value. The sequence then goes to step S29. Step S29 is a blood component concentration computation step. This step computes blood glucose concentration. This completes the object measurement step (step S3).

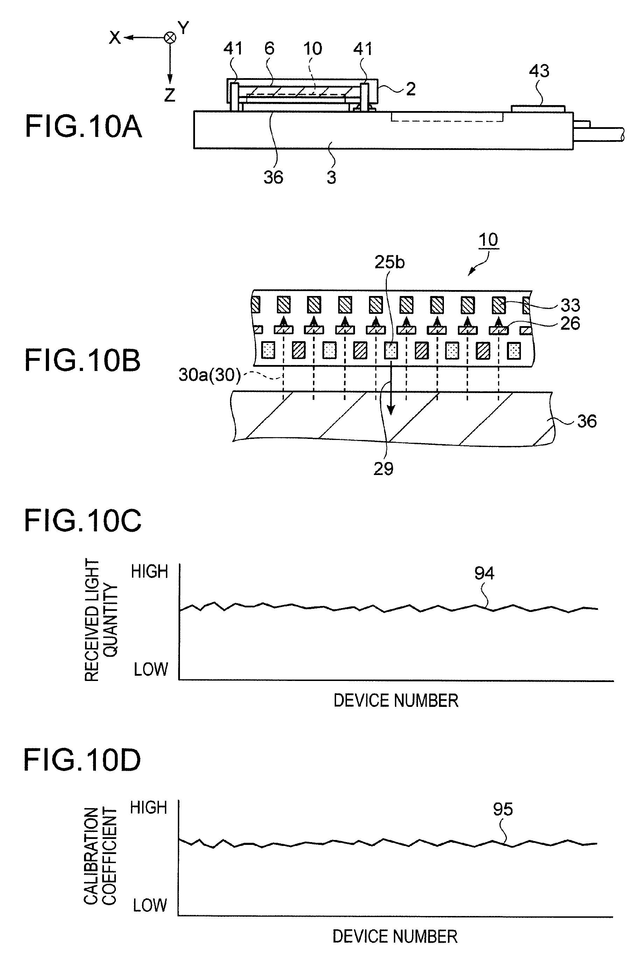

FIG. 10A to FIG. 14D are schematic views explaining the biological information acquisition method. Referring to FIG. 10A to FIG. 14D, the biological information acquisition method is described below in detail, along with the corresponding steps described in FIGS. 7 to 9. The sequence begins with the unit contacting step (step S11) in the maintenance step (step S1). FIG. 10A is a diagram corresponding to the unit contacting step (step S11). As represented in FIG. 10A, an operator in step S11 installs the first unit 2 on the second unit 3. The first unit 2 is installed using the locating projections 41 of the second unit 3 as a guide. This installs the sensor module 10 at a location opposite the calibration plate 36. The power connector 12 contacts the power socket 38. The communication connector 11 contacts the communication socket 37.

The operator operates the operation switches 43 to start the maintenance procedure. This starts the charging step (step S18). The second unit 3 supplies power to the first unit 2. In response, the rechargeable battery 22 starts charging in the first unit 2. In the second unit 3, the charge circuit 79 detects the charge state, and displays in the second display section 42 whether charging is in progress.

FIGS. 10B and 10C are diagrams corresponding to the calibration data acquisition step (step S12). As illustrated in FIG. 10B, in step S12, one of the measurement light-emitting devices 25b is turned on to irradiate the calibration plate 36. The measurement light 29 from the measurement light-emitting device 25b is reflected at the calibration plate 36, and becomes the second reflected light 30a. The second reflected light 30a off the calibration plate 36 irradiates the sensor module 10. The light-receiving devices 33 near the measurement light-emitting device 25b that has emitted light receive the second reflected light 30a, and detect its light intensity. Upon the light-receiving devices 33 detecting the light intensity, the measurement light-emitting device 25b is turned off, and another measurement light-emitting device 25b is turned on. In this manner, photodetection sensitivity data can be acquired for the combination of the activated measurement light-emitting device 25b and the light-receiving device 33.

The measurement light-emitting devices 25b are switched, and turned on one after another. The light-receiving devices 33 near the measurement light-emitting device 25b that has emitted light receive the second reflected light 30a, and detect its light intensity. In this manner, photodetection sensitivity data is acquired for all the measurement light-emitting devices 25b. In FIG. 10C, the vertical axis represents the light intensity detected by the light-receiving devices 33. The horizontal axis represents device number. The device number is a combination of numbers for the measurement light-emitting devices 25b and the light-receiving devices 33.

The measurement light-emitting devices 25b and the light-receiving devices 33 each have designated numbers. For example, the device number (2,5) is assigned to data detected by the fifth light-receiving device 33 from the light emitted by the second measurement light-emitting device 25b. A sensitivity data line 94 represents an example of light intensities for different device number combinations. As represented by the sensitivity data line 94, light intensities corresponding to combinations of measurement light-emitting devices 25b and light-receiving devices 33 are measured, and the measured data are stored as the calibration related data 86 in the second memory 77. The sensitivity data line 94, shown as a line chart, may be stored in a tabular form by tabulating device number and light intensity.

In the calibration coefficient computation step (step S13), the calibration data arithmetic section 91 computes the calibration coefficient. Prior to computation, a reference value of light intensity is set. Preferably, a reference value of light intensity is set using the light intensity received by a light-receiving device 33 of known performance under the measurement light 29 emitted by a measurement light-emitting device 25b of known performance.

The calibration data arithmetic section 91 then divides the reference value by the light intensity of each device number to calculate the calibration coefficient. The calibration coefficient is 1 when the reference value and the detected light intensity have the same value. The calibration coefficient becomes smaller than 1 when the detected light intensity is larger than the reference value. The calibration coefficient becomes larger than 1 when the detected light intensity is smaller than the reference value.

FIG. 10D is a diagram corresponding to the calibration coefficient computation step (step S13). In FIG. 10D, the vertical axis represents calibration coefficient. The horizontal axis represents device number. A calibration coefficient line 95 represents an example of calibration coefficients for different device number combinations. As represented by the calibration coefficient line 95, calibration coefficients corresponding to combinations of measurement light-emitting devices 25b and light-receiving devices 33 are computed, and the computed result is stored as the calibration related data 61 in the first memory 49. The calibration coefficient line 95, shown as a line chart, may be stored in a tabular form by tabulating device number and calibration coefficient.

In the measurement data transfer step (step S14), the first unit 2 transfers blood glucose concentration data to the second unit 3. Blood glucose concentration data are accumulated as the blood component value data 65 in the first memory 49 of the first unit 2. Blood glucose concentration data are data that have been measured in the object measurement step (step S3). Blood glucose concentration data are accumulated as the blood component value data 87 in the second memory 77 of the second unit 3. The blood glucose concentration data are transferred from the first memory 49 to the second memory 77. The amount of data in the first memory 49 decreases after the transfer, and the first memory 49 can be prevented from being overloaded. Blood glucose concentration data are accumulated in the second memory 77 every time the maintenance step (step S1) is performed. The second memory 77 can thus accumulate blood glucose concentration data over extended time periods.

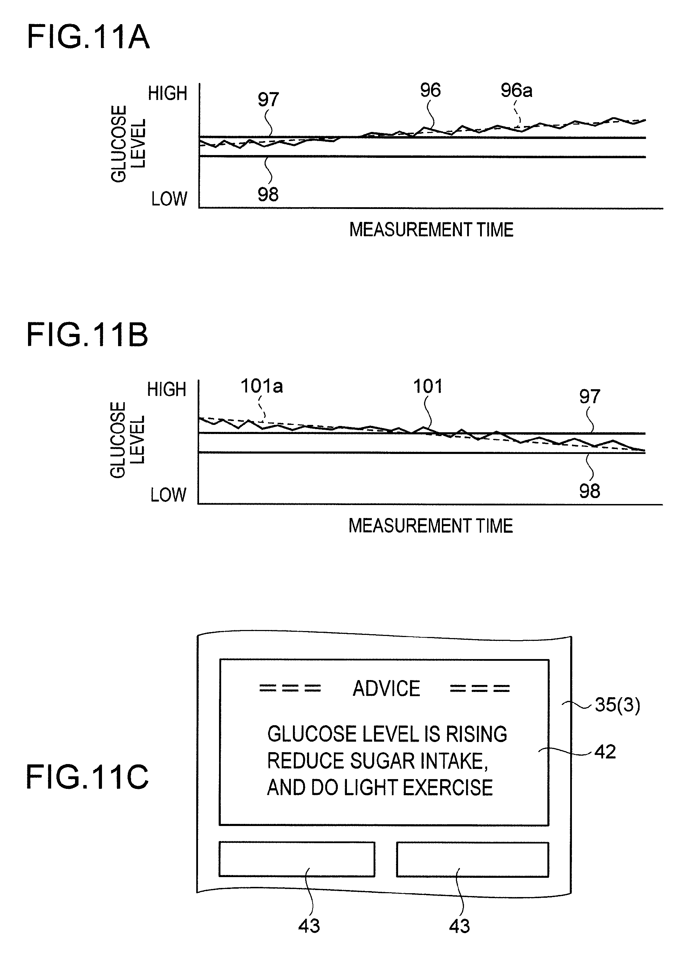

FIGS. 11A and 11B are diagrams corresponding to the measurement data analyzing step (step S15). As represented in FIG. 11A, blood glucose concentration changes are analyzed in step S15. In the figures, the vertical axis represents glucose level. The glucose level is higher from the bottom to top of the diagram. Glucose level is also referred to as blood glucose concentration. The horizontal axis represents measurement time. The direction of the passage of time is from right to left. A glucose level measurement line 96 represents an example of glucose level changes in the subject 4. The analysis arithmetic section 92 calculates a glucose level approximate line 96a from the glucose level measurement line 96 using the least squares approximation method. Whether the glucose level is rising or falling can be clearly found from the slope of the glucose level approximate line 96a. A rate of change also can be clearly found from the slope of the glucose level approximate line 96a.

The analysis arithmetic section 92 compares the glucose level approximate line 96a with an upper-limit determination value 97 and a lower-limit determination value 98. The glucose level is determined as normal when the glucose level approximate line 96a is at or below the upper-limit determination value 97, and is at or above the lower-limit determination value 98. The glucose level is determined as high when the glucose level approximate line 96a tends to be above the upper-limit determination value 97. The glucose level is determined as low when the glucose level approximate line 96a tends to be below the lower-limit determination value 98. In the example represented by the glucose level measurement line 96, it can be seen that the subject 4 has high glucose levels. The method used to determine glucose levels is not limited to this, and various other methods may be used.

FIG. 11B represents another example of glucose level changes in the subject 4. A glucose level measurement line 101 represents an example of glucose level changes in the subject 4. A glucose level approximate line 101a is an approximate line for the glucose level measurement line 101. As can be seen in the figure, the glucose level is normal because the glucose level approximate line 101a, initially above the upper-limit determination value 97, falls below the upper-limit determination value 97 and remains above the lower-limit determination value 98.

In the countermeasure selecting step (step S16), whether the glucose level is above or below the upper-limit determination value 97 and the lower-limit determination value 98 is determined by referring to the slope of the glucose level approximate line. How to deal with high sugar levels and low sugar levels is stored in the countermeasure data 89 of the second memory 77. From the countermeasure list in the countermeasure data 89, the countermeasure selecting section 93 selects a countermeasure that is considered to be most appropriate.

The countermeasures in the countermeasure data 89 are indexed, and are prepared according to the extent of high and low sugar levels. This enables the countermeasure selecting section 93 to easily select a countermeasure using the slope of the glucose level approximate line of the subject 4, and the results of comparisons with the upper-limit determination value 97 and the lower-limit determination value 98.

FIG. 11C is a diagram corresponding to the countermeasure display step (step S17). As represented in FIG. 11C, in step S17, the countermeasure selecting section 93 displays the glucose level status of the subject 4, and the selected countermeasure in the second display section 42. This completes the maintenance step (step S1), and the sequence goes to the unit mounting step (step S2).

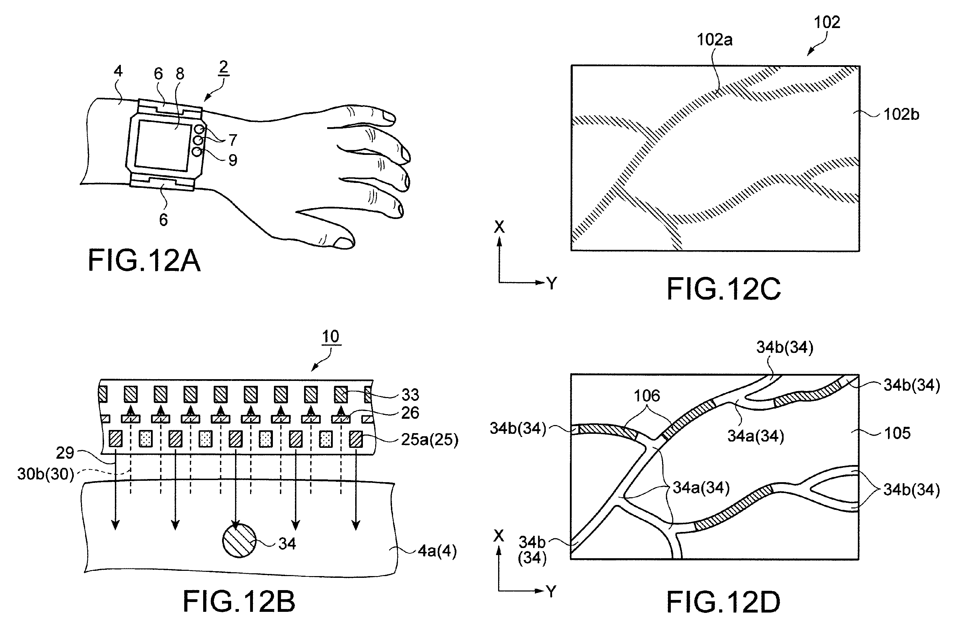

FIG. 12A is a diagram corresponding to the unit mounting step (step S2). As illustrated in FIG. 12A, the operator in step S2 installs the first unit 2 on the subject 4. The first unit 2 is installed in such a manner that the back surface 5b contacts the subject 4. Here, the first unit 2 is installed in such an orientation that the touch panel 8 can be seen. The operator presses the operation switches 7 to start a measurement, and the sequence goes to step S3.

The object measurement step (step S3) begins with step S21. FIGS. 12B and 12C are diagrams corresponding to the image acquisition step (step S21) . As illustrated in FIG. 12B, in step S21, an image of the measured portion 4a is captured. The biological image acquisition section 69 outputs to the emission control section 66 an instruction signal for turning on the imaging light-emitting devices 25a. The emission control section 66 outputs to the sensor drive circuit 50 the instruction signal for turning on the imaging light-emitting devices 25a. The sensor drive circuit 50 drives and turns on the imaging light-emitting devices 25a. The measurement light 29 emitted by the imaging light-emitting devices 25a irradiates the measured portion 4a. The measurement light 29 is reflected at the measured portion 4a, and becomes first reflected light 30b. The reflected light 30 off the measured portion 4a is referred to as first reflected light 30b.

The biological image acquisition section 69 outputs to the filter control section 68 an instruction signal for instructing the spectral devices 26 to pass light of 800 nm wavelength. The filter control section 68 outputs to the sensor drive circuit 50 an instruction signal for varying the wavelength characteristics of the spectral devices 26. The sensor drive circuit 50 drives the spectral devices 26, and sets an 800 nm wavelength for passage of light through the spectral devices 26. In this way, the first reflected light 30b of a wavelength that is easily absorbable by the blood vessel 34 passes through the spectral device 26, and it becomes easier to capture an image of the blood vessel 34.

The biological image acquisition section 69 outputs to the photoreception control section 67 an imaging instruction signal. The photoreception control section 67 outputs to the sensor drive circuit 50 an instruction signal for driving the light-receiving devices 33. The sensor drive circuit 50 drives the light-receiving devices 33, and outputs the light intensity of the input light to the photoreception control section 67 after converting the light intensity into photoreception data. By being arrayed in a grid, the light-receiving devices 33 function as an image capturing camera. The photoreception data forms a biological image 102 representing the captured shape of the blood vessel 34, as shown in FIG. 12C. The photoreception control section 67 stores the biological image 102 as the biological image data 58 in the first memory 49.

FIG. 12C is a diagram corresponding to the image acquisition step (step S21) and the blood vessel location acquisition step (step S22). The biological image 102 shown in FIG. 12C is an output image of the measured portion 4a from the sensor module 10. The biological image 102 is obtained as a two-dimensional image with pixels corresponding to the array of the light-receiving devices 33 in the sensor module 10. The blood vessel 34 more easily absorbs near-infrared rays than the non-blood vessel portion. Accordingly, the blood vessel image 102a, an image of the blood vessel 34, has lower luminance, and appears darker than the non-blood vessel image 102b of the non-blood vessel portion in the biological image 102. A blood vessel pattern can thus be extracted by extracting the lower luminance portion in the biological image 102 in step S22. Specifically, the presence or absence of the blood vessel 34 directly below the light-receiving device 33 can be determined by determining whether the luminance of the corresponding pixel constituting the biological image 102 has a value that is equal to or less than a predetermined threshold value. This makes it possible to detect the location of the blood vessel 34.

FIG. 12D is a diagram corresponding to the measurement target selecting step (step S23), schematically representing blood vessel location information obtained from the biological image 102. The blood vessel location information is information indicative of whether the location corresponding to each pixel of the biological image 102 is the blood vessel 34 or the non-blood vessel portion 105. In step S23, the measurement location arithmetic section 70 selects a measurement site 106, a measurement location of the blood vessel 34. The measurement location arithmetic section 70 selects the measurement site 106 by satisfying the following selection conditions. The measurement site 106 satisfies the selection conditions when it is not a branching or a merging portion of the blood vessel, or an end portion of the image, and has a predetermined length and width.

At branching and merging portions 34a of the blood vessel, the reflected light 30 has the possibility of mixing with light that has passed through a blood vessel 34 that is not a measurement target. The light that has passed through a blood vessel 34 that is not a measurement target has the possibility of affecting the absorption spectrum of the measurement site 106 selected as the measurement target. This may result in poor measurement accuracy. The measurement site 106 is thus selected from portions other than the branching and merging portions 34a of the blood vessel 34.

At end portions 34b of the blood vessel 34 in the biological image 102, there is no information about the blood vessel structure in the vicinity of the outer side of the image, whether the blood vessel is branched or merging. For the same reason described above, the measurement site 106 is thus selected from portions of blood vessel 34 other than the end portions 34b of the biological image 102 to avoid the possibility of lowering measurement accuracy.

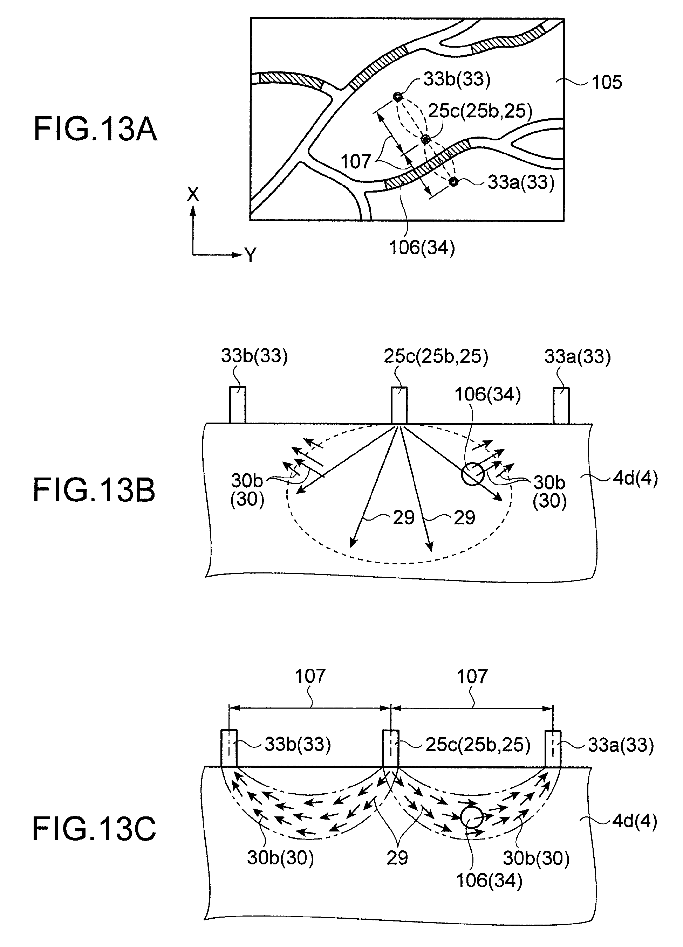

FIG. 13A is a diagram corresponding to the light-emitting and light-receiving device selecting step (step S24). As illustrated in FIG. 13A, the measurement location arithmetic section 70 in step S24 selects a measurement light-emitting device 25b and a light-receiving device 33 that are to be driven for measurement. Here, a measurement light-emitting device 25b and a light-receiving device 33 are selected so that the measurement site 106 is between the measurement light-emitting device 25b and the light-receiving device 33. The light-receiving device 33 detects light that has passed through the measurement site 106.

The measurement location arithmetic section 70 also selects a measurement light-emitting device 25b and a light-receiving device 33 that are to be driven for reference measurement. Here, a measurement light-emitting device 25b and a light-receiving device 33 are selected so that the measurement site 106 is not between the measurement light-emitting device 25b and the light-receiving device 33. The light-receiving device 33 detects light that did not pass through the measurement site 106. This measurement will be referred to as reference measurement. In the present embodiment, the same device is set for the measurement light-emitting device 25b and the reference measurement light-emitting device 25 at the same location.

Assume here that the measurement light-emitting device 25b at the irradiation position is a light-emitting device 25c, and the light-receiving device 33 at the reception position for measurement is a measurement light-receiving device 33a. The measurement location arithmetic section 70 sets locations for the light-emitting device 25c and the measurement light-receiving device 33a so that the measurement site 106 is centered between the light-emitting device 25c and the measurement light-receiving device 33a. The measurement location arithmetic section 70 also sets locations for the light-emitting device 25c and the measurement light-receiving device 33a so that the distance between the light-emitting device 25c and the measurement light-receiving device 33a becomes a predetermined optimum distance 107.

Assume here that the light-receiving device 33 at the reference reception position for reference measurement is a reference light-receiving device 33b. The light-emitting device 25 at the irradiation position for reference measurement is the light-emitting device 25c. The location for the reference light-receiving device 33b is set so that the blood vessel 34 does not exist between the light-emitting device 25c and the reference light-receiving device 33b. The measurement location arithmetic section 70 sets the locations for the light-emitting device 25c and the reference light-receiving device 33b so that the distance between the light-emitting device 25c and the reference light-receiving device 33b becomes the predetermined optimum distance 107.

FIGS. 13B and 13C are diagrams corresponding to the measurement step (step S25). These are schematic cross sectional views taken in depth direction, explaining propagation of light inside the body tissue. Hatching is omitted for viewability. As illustrated in FIG. 13B, the light-emitting device 25c in step S25 emits measurement light 29 in a predetermined directional characteristic. The cellular tissue surrounding the blood vessel 34 in the subject 4 represents a common tissue 4d. The common tissue 4d is a cellular tissue including, for example, skin tissue, adipose tissue, and muscle tissue, surrounding the blood vessel 34 being measured. Some of the measurement light 29 passes through the blood vessel 34 through the common tissue 4d. Some of the measurement light 29 passes through the blood vessel 34 after being scattered by the common tissue 4d. Some of the measurement light 29 passes through the blood vessel 34, and enter the measurement light-receiving device 33a as first reflected light 30b. Some of the measurement light 29 enters the measurement light-receiving device 33a and the reference light-receiving device 33b as first reflected light 30b, without passing through the blood vessel 34.

FIG. 13C is a diagram simulating the paths of light rays emitted by the light-emitting device 25 and entering the light-receiving devices 33, using a ray tracing method. As illustrated in FIG. 13C, the measurement light 29 radiating from the light-emitting device 25c undergoes diffuse reflection inside the body tissue, and some of the radiating light reaches the light-receiving devices 33. The light paths of the propagating light travel through banana-shaped regions confined between two arcs. The light path is widest along the depth direction near substantially the center between the light-emitting device 25 and the light-receiving device 33. The light path is also deepest in this part of the tissue. The reachable light depth increases as the distance between the light-emitting device 25 and the light-receiving device 33 increases.

For improved measurement accuracy, it is desirable that the light-receiving device 33 receives more transmitted light from the blood vessel 34. For this reason, it is desirable to locate the measurement target, or the measurement site 106, at substantially the center between the light-emitting device 25 and the light-receiving device 33. The optimum distance 107 is specified according to the supposed depth of the measurement site 106. The optimum distance 107 representing the optimum interval between the light-emitting devices 25 and the light-receiving devices 33 is about two times the depth of the blood vessel 34 from skin surface. For example, the optimum distance 107 is about 5 to 6 mm for a depth of about 3 mm.

The wavelength of the measurement light 29 emitted by the light-emitting device 25c is such that the absorbance varies with blood glucose levels. Some of the reflected light 30 detected by the measurement light-receiving device 33a passes through the blood vessel 34, and some of the first reflected light 30b is absorbed by blood in the blood vessel 34. Accordingly, the output data from the measurement light-receiving device 33a contains information about the blood absorbance and the absorbance of the non-blood vessel portion 105. On the other hand, the reflected light 30 detected by the reference light-receiving device 33b does not pass through the blood vessel 34, and is not absorbed by blood in the blood vessel 34. Accordingly, the output data from the reference light-receiving device 33b contains information about the absorbance of the non-blood vessel portion 105.

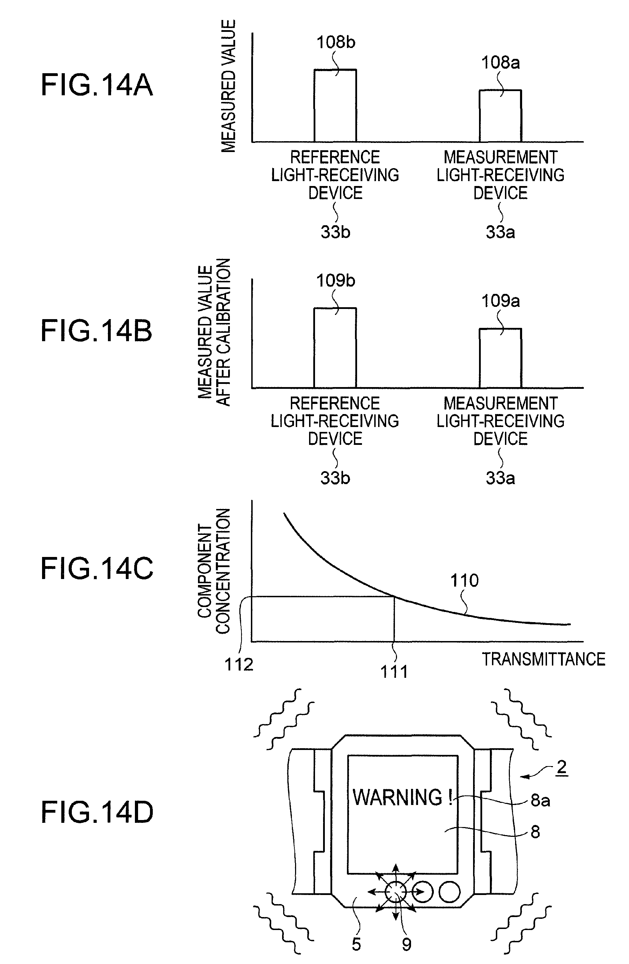

FIGS. 14A and 14B are diagrams corresponding to the calibration step (step S26). In FIG. 14A, the vertical axis represents measured value, specifically the light intensity value detected by the light-receiving device 33. The light intensity on vertical axis becomes higher from the bottom to top. The horizontal axis depicts the measurement light-receiving device 33a and the reference light-receiving device 33b. The measured values by the measurement light-receiving device 33a and the reference light-receiving device 33b are presented as a bar chart. The measured values detected by the measurement light-receiving device 33a and the reference light-receiving device 33b are given as blood measurement value 108a and reference measurement value 108b, respectively.

The calibration data arithmetic section 91 multiplies the measured value by the calibration coefficient. The calibration coefficient is the coefficient calculated by the calibration data arithmetic section 91 in step S1. The calibration coefficient is set for each combination of the light-emitting device 25 and the light-receiving device 33. In FIG. 14B, the vertical axis represents measured value after calibration, specifically value after the calibration of the light intensity value detected by the light-receiving device 33. The light intensity on vertical axis becomes higher from the bottom to top. The horizontal axis depicts the measurement light-receiving device 33a and the reference light-receiving device 33b. The calibrated blood measurement value 109a and the calibrated reference measurement value 109b are presented as a bar chart.

In this step, the blood measurement value 108a is multiplied by the calibration coefficient corresponding to the combination of the light-emitting device 25c and the measurement light-receiving device 33a to calculate the calibrated blood measurement value 109a. The calibrated reference measurement value 109b is calculated by multiplying the reference measurement value 108b by the calibration coefficient corresponding to the combination of the light-emitting device 25c and the reference light-receiving device 33b.

The light-emitting devices 25 and the light-receiving devices 33 have performance variance attributed to production. There is also a performance change due to changes with time. In step S1, the calibration coefficient is set with the use of the calibration plate 36 having a reflectance that is uniform throughout the plane and that does not easily undergo changes with time. In step S26, the measured values are calibrated with the calibration coefficient. The calibrated blood measurement value 109a and the calibrated reference measurement value 109b obtained in step S26 are thus unlikely to be affected by changes occurring in the light-emitting devices 25 and the light-receiving devices 33 over time, or by the production variance of the light-emitting devices 25 and the light-receiving devices 33.

In the absorption spectrum computation step (step S27) , the transmittance through the blood vessel 34 is computed with the calibrated blood measurement value 109a and the calibrated reference measurement value 109b. The transmittance may be calculated through four arithmetic operations of the calibrated blood measurement value 109a and the calibrated reference measurement value 109b. In a simpler operation, the calibrated blood measurement value 109a may be divided by the calibrated reference measurement value 109b to obtain a transmittance. The operation of the calibrated blood measurement value 109a may take into account the proportion of the first reflected light 30b that passed through the blood vessel 34. The proportion of the first reflected light 30b that passed through the blood vessel 34 may be calculated using methods such as a phantom method, and a Monte Carlo simulation method.

In the average absorption spectrum computation step (step S28), the mean value is computed using a plurality of transmittance values. Step S25 has been described through the case of a measurement at a single measurement location. The mean value is computed in step S28 when there is more than one measurement location. The moving average may be computed when performing measurements at predetermined time intervals. Step S28 may be omitted when the mean is not computed.

FIG. 14C is a diagram corresponding to the blood component concentration computation step (step S29). In step S29, the calculated transmittance is used to compute blood glucose concentration. In FIG. 14C, the vertical axis represents blood glucose concentration. The concentration is higher from the bottom to top of the diagram. The horizontal axis represents transmittance, representing the blood transmittance rate of light of the same wavelength as the wavelength of the measurement light 29. In the diagram, the transmittance increases from the left to right. A correlation curve 110 represents the relationship between blood transmittance and blood glucose concentration. Absorption of light increases with increase of blood glucose concentration, and the transmittance decreases. When the mean value calculated in the step S28 is a calculated transmittance value 111, the correlation curve 110 is used to calculate an arithmetic concentration value 112 representing blood glucose concentration. The correlation curve 110 may be represented as a function, or as a correlation table in a tabular form. The arithmetic concentration value 112 can be calculated from the calculated transmittance value 111 also in these cases. This completes the object measurement step (step S3), and the sequence goes to step S4.

In the warning determination step (step S4), the arithmetic concentration value 112 is compared to determination values. The determination values include an upper determination value and a lower determination value. The current state is determined as normal, and not in need of a warning when the arithmetic concentration value 112 is at or below the upper determination value, and at or above the lower determination value. The current state is determined as abnormal when the arithmetic concentration value 112 is higher than the upper determination value. The current state is also determined as abnormal when the arithmetic concentration value 112 is below the lower determination value. In an abnormal state, it is determined to give a warning, and the sequence goes to step S5.

FIG. 14D is a diagram corresponding to the warning step (step S5). As illustrated in FIG. 14D, the subject 4 is warned in step S5. The touch panel 8 displays a warning text 8a. The warning text 8a contains a statement explaining that the subject 4 is at risk. The subject 4 reading the statement can easily understand his or her status. The speaker 9 produces a warning sound. Warning sound data are prestored in the first memory 49, and the first CPU 48 outputs to the speaker 9 a voltage waveform based on the warning sound data. The speaker 9 outputs sound after converting the voltage waveform into a sound wave. The subject 4 also can be brought to attention even when he or she is not looking at the touch panel 8. The first CPU 48 vibrates the first exterior portion 5 by driving the vibrator 16. Because the first exterior portion 5 is in contact with the subject 4, the vibration is transmitted to the subject 4. The subject 4 can then be brought to attention that he or she is in an abnormal state.

The maintenance determination step (step S6) determines whether to maintain the first unit 2. It is determined to maintain the first unit 2 when the accumulated power in the rechargeable battery 22 is below a determination value. This step also determines to perform maintenance when the stored data in the first memory 49 is approaching the allowable volume. It is also determined to perform maintenance when a predetermined time period has elapsed from the last time the maintenance step (step S1) was performed. The maintenance determination step determines not to perform maintenance when these conditions are not met. When it is determined to perform maintenance, the first unit 2 is removed from the subject 4, and the sequence goes to step S1. The sequence goes to step S7 when it is determined not to perform maintenance.

In the end determining step (step S7), it is determined whether to end the acquisition of blood glucose concentration information. It is determined to end the acquisition upon the operator operating the operation switches 7, the operation input section 24, or the operation switches 43, and giving an instruction to end the acquisition of blood glucose concentration information. It is determined to continue the process, and steps S3 to S7 are repeated when the operator does not give an instruction to end the acquisition. The object measurement step (step S3) is thus repeatedly performed with the first unit 2 installed on the subject 4. Blood glucose concentration changes in the subject 4 can be detected even when the subject 4 is moving.

When it is determined in step S7 to end the process, the first unit 2 is removed from the subject 4, and the sequence goes to step S8. The content of the maintenance step (step S8) is the same as that of step S1. As such, the rechargeable battery 22 is charged, and calibration data is computed. The blood component value data 65 in the first memory 49 are transferred to the second memory 77, and information of blood glucose concentration is analyzed. This completes the acquisition of glucose concentration information from the subject 4.

As described above, the present embodiment has the following effects.
(1) According to the present embodiment, the light-receiving devices 33 upon receiving the first reflected light 30b output a signal corresponding to the light intensity of the first reflected light 30b reflected at the measured portion 4a. Upon reflecting light, the measured portion 4a absorbs light of a specific wavelength that varies with the blood glucose concentration. Blood glucose concentration can thus be measured by analyzing the output light intensity of the first reflected light 30b from the light-receiving devices 33.
(2) According to the present embodiment, the component measurement apparatus 1 includes the first unit 2 and the second unit 3. The first unit 2 and the second unit 3 are separable from each other. This enables saving the weight of the first unit 2. The first unit 2 is used by being mounted on the subject 4. With the lightness of the unit mounted on the subject 4, the component measurement apparatus 1 can measure glucose levels with good portability.
(3) According to the present embodiment, the second unit 3 includes the calibration plate 36 with which the second reflected light 30a for comparing the light intensity of the first reflected light 30b is output to the light-receiving devices 33. Upon receiving the second reflected light 30a, the light-receiving devices 33 output a signal corresponding to the light intensity of the second reflected light 30a at the calibration plate 36. The performance of the light-emitting devices 25 that apply light to the calibration plate 36 and the measured portion 4a changes over time. The rate at which the light-receiving devices 33 convert the reflected light 30 into a signal also changes with time. On the other hand, the reflectance of the calibration plate 36 remains stable for extended time periods. Changes in the detected light intensity of the second reflected light 30a at the calibration plate 36 have a correlation with the effects of changes occurring in the performance of the light-emitting devices 25 and the light-receiving devices 33. Changes in the detected light intensity of the second reflected light 30a at the calibration plate 36, and the detected light intensity of the first reflected light 30b at the measured portion 4a can thus be used to accurately detect the characteristics of the first reflected light 30b at the measured portion 4a.
(4) According to the present embodiment, the first unit 2 has the locating receptacles 5c in the first exterior portion 5, and the second unit 3 has the locating projections 41. The sensor module 10 and the calibration plate 36 are oriented face to face with the locating receptacles 5c and the locating projections 41. This ensures that the light-receiving devices 33 receive the second reflected light 30a reflected at the calibration plate 36 upon application of the light by the light-emitting devices 25.
(5) According to the present embodiment, the light-emitting devices 25 and the light-receiving devices 33 have optical axes in the same direction. In the sensor module 10, the direction with a high emission quantity and the direction with the highest photoreception sensitivity are the same. The sensor module 10 can thus receive the second reflected light 30a with good sensitivity with the calibration plate 36 installed in the direction of the optical axes of the light-emitting devices 25 and the light-receiving devices 33. Likewise, the sensor module 10 can receive the first reflected light 30b with good sensitivity with the measured portion 4a placed in the direction of the optical axes of the light-emitting devices 25 and the light-receiving devices 33.
(6) According to the present embodiment, the calibration plate 36 contains polytetrafluoroethylene. Polytetrafluoroethylene reflects near-infrared light without absorbing it. This makes it possible to efficiently obtain the second reflected light 30a used for calibration.
(7) According to the present embodiment, the component measurement apparatus 1 includes the component value calculating section 73, the abnormal state determining section 74, and the speaker 9. The component value calculating section 73 computes a glucose level using an output signal corresponding to the light intensity of the first reflected light 30b from the photoreceiver. The determining section compares the glucose level with a determination value to determine whether the object is in an abnormal state. The speaker 9 gives a warning when it is determined that the subject 4 is in an abnormal state. This makes it possible to immediately notify the subject 4 of an abnormal state when the subject 4 is in an abnormal state.
(8) According to the present embodiment, the first unit 2 includes the first communication section 51, and the second unit 3 includes the second communication section 78. The first unit 2 sends glucose level information to the second unit 3. The second unit 3 includes the second memory 77, and the blood component value data 87 are stored in the second memory 77. The second memory 77 can store long-term information concerning the blood component value data 87. This makes it possible to analyze information of changing glucose levels over extended time periods.
(9) According to the present embodiment, the analysis arithmetic section 92 analyzes information of the blood component value data 87. The second memory 77 of the second unit 3 stores long-term information of glucose levels. The analysis arithmetic section 92 can thus analyze long-term patterns of glucose levels, and long periodic changes of glucose levels.
(10) According to the present embodiment, the analysis arithmetic section 92 selects a countermeasure for the subject 4 from the countermeasure data 89. The second display section 42 displays the countermeasure. The subject 4 can thus recognize ways to maintain normal glucose levels.
(11) According to the present embodiment, the light intensity detection of the first reflected light 30b, and the computation of blood glucose concentration are repeated with the component measurement apparatus 1 installed on the subject 4. Blood glucose concentration changes in the subject 4 can thus be detected even when the subject 4 is moving.

### Second Embodiment

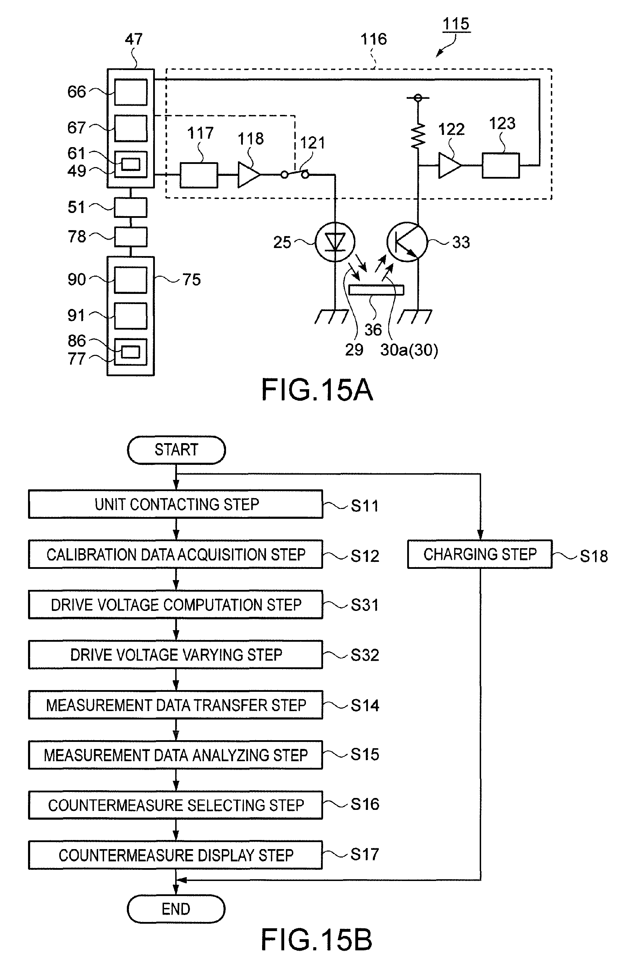

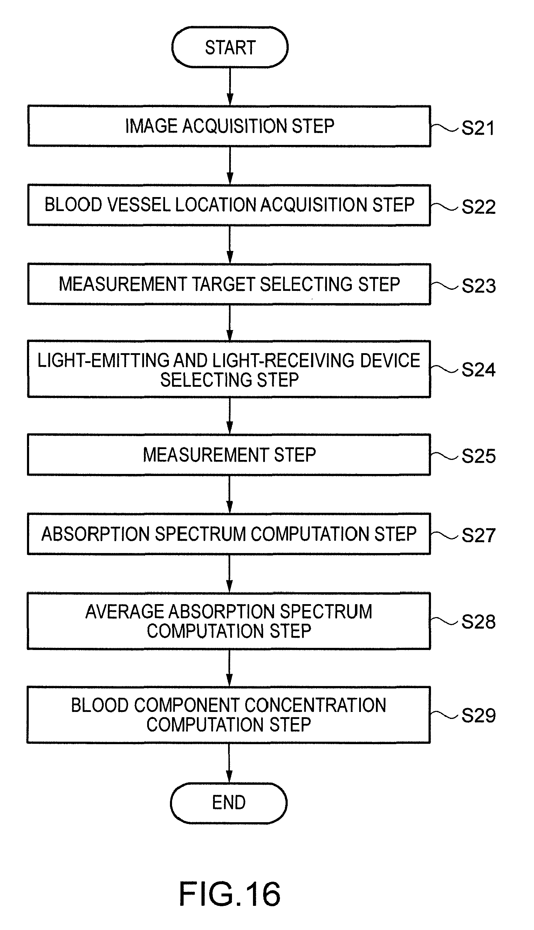

An embodiment of the component measurement apparatus is described below with reference to FIGS. 15A and 15B, and FIG. 16. FIG. 15A is a block diagram representing a relevant portion of a sensor drive circuit according to Second Embodiment, and FIG. 15B is a flowchart representing a maintenance step (step S1) in detail. FIG. 16 is a flowchart representing an object measurement step (step S3) in detail. The present embodiment differs from First Embodiment in that the value measured with the calibration plate 36 is used to adjust the output of the light-emitting device 25. The same features already described in First Embodiment will not be described further.

Specifically, in the present embodiment, a sensor drive circuit 116 connected to the first controller 47 is installed in a component measurement apparatus 115 (information acquisition apparatus), as shown in FIG. 15A. The sensor drive circuit 116 drives the light-emitting devices 25, the spectral devices 26, and the light-receiving devices 33. The first controller 47 has an emission control section 66, and a photoreception control section 67 to realize its functions. The first controller 47 also has a region in first memory 49 where calibration related data 61 are stored.

The second controller 75 has a calibration measurement control section 90 and a calibration data arithmetic section 91 to realize its functions. The second controller 75 also has a region in second memory 77 where calibration related data 86 are stored. The first controller 47 and the second controller 75 communicate with each other via the first communication section 51 and the second communication section 78.

The sensor drive circuit 116 includes a first D/A (Digital/Analog) converter 117, a first amplifier 118, and a switch section 121. The first controller 47 and the first D/A converter 117 are connected to each other, and the first D/A converter 117, the first amplifier 118, and the switch section 121 are connected in this order. The switch section 121 is connected to the light-emitting device 25. The first D/A converter 117, the first amplifier 118, and the switch section 121 are provided in the same number as the number of light-emitting devices 25. A different applied voltage may be set for each different light-emitting device 25. The sensor drive circuit 116 also includes a second amplifier 122, and an A/D (Analog/Digital) converter 123. The light-receiving devices 33, the second amplifier 122, the A/D converter 123, and the first controller 47 are connected in this order.

The calibration related data 61 includes drive voltage data for driving the light-emitting devices 25. The calibration measurement control section 90 outputs light-emitting and light-receiving instruction signals to the emission control section 66 and the photoreception control section 67. The emission control section 66 receives drive voltage data for the light-emitting device 25 from the calibration related data 61, and outputs the data to the first D/A converter 117. The first D/A converter 117 converts the voltage data into a voltage signal, and outputs the signal to the first amplifier 118. The first amplifier 118 receives the voltage data, and outputs it to the switch section 121 after amplifying the power. The switch section 121 receives the instruction signal from the emission control section 66, and the power amplified voltage signal. The switch section 121 then outputs to the light-emitting device 25 a voltage waveform corresponding to the instruction signal. This drives the light-emitting device 25 according to the voltage instructed by the emission control section 66. The light-emitting device 25 emits the measurement light 29. The measurement light 29 is applied to the calibration plate 36.

The second reflected light 30a reflected at the calibration plate 36 enters the light-receiving device 33. The light-receiving device 33 converts the light intensity of the second reflected light 30a into voltage, and outputs the voltage signal to the second amplifier 122. The second amplifier 122 amplifies the input voltage signal, and outputs it to the A/D converter 123. The A/D converter 123 converts the voltage signal into voltage data, and outputs it to the first controller 47. In the first controller 47, the first CPU 48 sends the corresponding voltage data of the second reflected light 30a to the second controller 75, and the data are stored in the second memory 77.

In FIG. 15B, steps S11 and S12 are the same as in First Embodiment, and will not be described. The sequence goes to step S31 after step S12. In the drive voltage computation step (step S31), the calibration data arithmetic section 91 computes a drive voltage for the light-emitting device 25. Prior to computation, a reference value is set for the voltage corresponding to the light intensity received by the light-receiving device 33. The reference value includes an upper-limit reference value corresponding to the upper-limit light intensity, and a lower-limit reference value corresponding to the lower-limit light intensity. The calibration data arithmetic section 91 receives from the second memory 77 the voltage data corresponding to the second reflected light 30a detected in step S12.

The calibration data arithmetic section 91 then compares the corresponding voltage data of the second reflected light 30a with the reference value. The drive voltage data driving the light-emitting device 25 is decreased when the voltage data exceeds the upper-limit reference value. The drive voltage data driving the light-emitting device 25 is increased when the voltage data is below the lower-limit reference value. The drive voltage data is varied over a range that is proportional to the difference between the voltage data and the reference value. The calibration data arithmetic section 91 compares the corresponding voltage data of the second reflected light 30a with the reference value for all the light-emitting devices 25, and varies the drive voltage data when the voltage data is larger than the upper-limit reference value and when the voltage data is smaller than the lower-limit reference value. The varied data is stored in the calibration related data 86 in the second memory 77. The sequence then goes to step S32.

In the drive voltage varying step (step S32), the drive voltage data varied in step S31 is transferred from the second memory 77 to the first memory 49. This varies the drive voltage data stored in the first memory 49. The sequence then goes to step S14. Steps S14 to S17 are the same as in First Embodiment, and will not be described. The following describes the object measurement step (step S3).

In FIG. 16, steps S21 to S25 are the same as in First Embodiment, and will not be described. The sequence goes to step S27 (absorption spectrum computation step) after step S25. The calibration step (step S26) is omitted. Step S26 can be omitted because the drive voltage for the light-emitting device 25 is varied in steps S31 and S32. Steps S27 to S29 are the same as in First Embodiment, and will not be described.

As described above, the present embodiment has the following effects.
(1) According to the present embodiment, the voltage driving the light-emitting device 25 is calibrated when there is a performance change in the light-emitting devices 25 and the light-receiving devices 33. The output voltage data from the sensor drive circuit 116 to the first controller 47 can thus accurately reflect the state of the measured portion 4a.
(2) According to the present embodiment, the voltage driving the light-emitting device 25 is increased when there is a performance drop in the light-emitting devices 25 and the light-receiving devices 33. This increases the light intensity of the measurement light 29, and can suppress decrease of the SN ratio (Signal Noise) in the output voltage data to the first controller 47.

### Third Embodiment

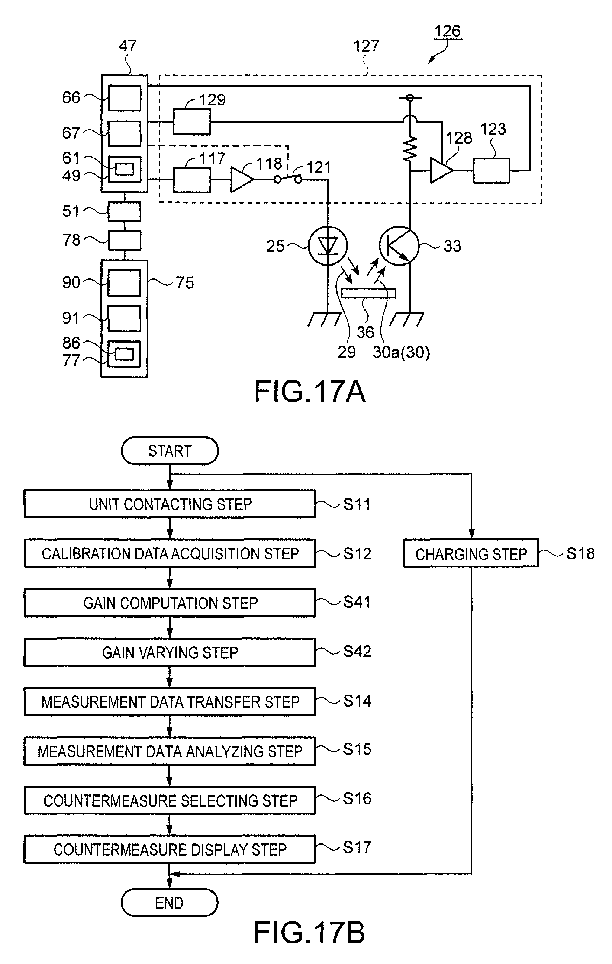

An embodiment of the component measurement apparatus is described below with reference to FIGS. 17A and 17B. FIG. 17A is a block diagram representing a relevant portion of a sensor drive circuit. FIG. 17B is a flowchart representing a maintenance step (step S1) in detail. The present embodiment differs from Second Embodiment in that the value measured with the calibration plate 36 is used to adjust the amplification gain for the output of the light-receiving device 33. The same features already described in First and Second Embodiments will not be described further.

Specifically, in the present embodiment, a sensor drive circuit 127 connected to the first controller 47 is installed in a component measurement apparatus 126 (information acquisition apparatus), as shown in FIG. 17A. The sensor drive circuit 127 drives the light-emitting devices 25, the spectral devices 26, and the light-receiving devices 33. The first controller 47 has an emission control section 66, and a photoreception control section 67 to realize its functions. The first controller 47 also has a region in first memory 49 where calibration related data 61 are stored.

The second controller 75 has a calibration measurement control section 90, and a calibration data arithmetic section 91 to realize its functions. The second controller 75 also has a region in second memory 77 where calibration related data 86 are stored. The first controller 47 and the second controller 75 communicate with each other via the first communication section 51 and the second communication section 78.

The sensor drive circuit 127 includes a first D/A converter 117, a first amplifier 118, and a switch section 121. The first controller 47 and the first D/A converter 117 are connected to each other, and the first D/A converter 117, the first amplifier 118, and the switch section 121 are connected in this order. The switch section 121 is connected to the light-emitting device 25. The first D/A converter 117, the first amplifier 118, and the switch section 121 are provided in the same number as the number of light-emitting devices 25. A different applied voltage may be set for each different light-emitting device 25. The sensor drive circuit 127 also includes a second amplifier 128, a second D/A converter 129, and an A/D converter 123. The light-receiving device 33, the second amplifier 128, the A/D converter 123, and the first controller 47 are connected in this order. The second amplifier 128 has a variable gain, and is connected to the first controller 47 via the second D/A converter 129.

The light-emitting devices 25 apply the measurement light 29 to the calibration plate 36. The second reflected light 30a reflected at the calibration plate 36 enters the light-receiving device 33. The light-receiving device 33 converts the light intensity of the second reflected light 30a into voltage, and outputs the voltage signal to the second amplifier 128. The second amplifier 128 amplifies the input voltage signal, and outputs it to the A/D converter 123. The A/D converter 123 converts the voltage signal into voltage data, and outputs it to the first controller 47. In the first controller 47, the first CPU 48 sends the corresponding voltage data of the second reflected light 30a to the second controller 75, and the data are stored in the second memory 77.

The calibration related data 61 includes gain data for the second amplifier 128. The photoreception control section 67 outputs the gain data to the second D/A converter 129. The second D/A converter 129 converts the gain data into a voltage signal indicative of a gain, and outputs it to the second amplifier 128. The second amplifier 128 receives the voltage signal indicative of a gain, and amplifies the corresponding voltage signal of the second reflected light 30a with the instructed gain. The second amplifier 128 amplifies the input voltage signal, and outputs it to the A/D converter 123.

In FIG. 17B, steps S11 and S12 are the same as in First Embodiment, and will not be described. The sequence goes to step S41 after step S12. In the gain computation step (step S41), the calibration data arithmetic section 91 computes a gain of the second amplifier 128. Prior to computation, a reference value is set for the voltage corresponding to the light intensity received by the light-receiving device 33. The reference value includes an upper-limit reference value corresponding to the upper-limit light intensity, and a lower-limit reference value corresponding to the lower-limit light intensity. The calibration data arithmetic section 91 receives from the first memory 49 the voltage data corresponding to the second reflected light 30a detected in step S12.

The calibration data arithmetic section 91 then compares the corresponding voltage data of the second reflected light 30a with the reference value. The gain data indicative of the gain of the second amplifier 128 is decreased when the voltage data exceeds the upper-limit reference value. The gain data is increased when the voltage data is below the lower-limit reference value. The gain data is varied over a range that is proportional to the difference between the voltage data and the reference value. As a result, the voltage data corresponding to the second reflected light 30a takes a value between the upper-limit reference value and the lower-limit reference value. The calibration data arithmetic section 91 compares the corresponding voltage data of the second reflected light 30a with the reference value for all the light-receiving devices 33, and varies the gain data when the voltage data is larger than the upper-limit reference value and when the voltage data is smaller than the lower-limit reference value. In this manner, the gain data is varied so that the voltage data corresponding to the second reflected light 30a takes the same value as the reference value in all the light-receiving devices 33. The sequence then goes to step S42.

In the gain varying step (step S42), the gain data varied in step S41 is transferred in the first memory 49. This varies the gain data stored in the first memory 49. The sequence then goes to step S14. Steps S14 to S17 are the same as in First Embodiment, and will not be described. In the object measurement step (step S3), the calibration step (step S26) is omitted, as in Second Embodiment.

As described above, the present embodiment has the following effect.
(1) According to the present embodiment, the gain of the second amplifier 128 is varied when there is a performance change in the light-emitting devices 25 and the light-receiving devices 33. The output voltage data from the sensor drive circuit 127 to the first controller 47 can thus accurately reflect the state of the measured portion 4a.

The present embodiment is not limited to the description of the embodiments above, but may be altered or modified in many ways by a person with ordinary skill in the art within the technical idea of the invention. Variations are described below.

### Variation 1

In the foregoing First Embodiment, the computed blood component is glucose concentration. However, this should not be construed as a limitation, and blood oxygen concentration may be measured using the transmittance of hemoglobin. Hemoglobin can be detected with measurement light 29 of about 650 nm wavelength. A wavelength of about 650 nm is thus set for passage of reflected light 30 through the spectral devices 26. The transmittance can then be computed to measure blood oxygen concentration. Aside from blood oxygen concentration, the concentration of other components such as lipids may be computed. The blood vessels are not a limitation, and the concentration of the lymph fluid component in a lymph duct may be measured and computed. It is also possible to measure and compute the concentration of the cerebrospinal fluid component. The component measurement apparatus 1 also may be used to test animals other than humans. Aside from animals, the component measurement apparatus 1 also may be used for the measurement of the liquid components or concentrations in plants such as fruits.

### Variation 2

In the foregoing First Embodiment, the light-emitting devices 25 are installed in the sensor module 10. However, the light-emitting devices 25 may be excluded from the sensor module 10, and the measurement light 29 may be applied to the measured portion 4a from a light source different from the light-emitting devices 25. Because the light-emitting devices 25 are absent, the sensor module 10 can be produced with improved productivity.

### Variation 3

In the foregoing First Embodiment, the second unit 3 performs the functions of the calibration measurement control section 90 and the calibration data arithmetic section 91. However, the functions of the calibration measurement control section 90 and the calibration data arithmetic section 91 may be performed by the first unit 2. In this way, the communication volume between the first unit 2 and the second unit 3 can be reduced. This makes it possible to reduce the time required for the maintenance step (step S1).

### Variation 4

In the foregoing Second Embodiment, the input voltage signal to the first amplifier 118 from the first D/A converter 117 is varied. However, it is also possible to vary the gain of the first amplifier 118, as in Third Embodiment. The light intensity of the measurement light 29 can also be varied in this manner.

### Variation 5

In the foregoing Second Embodiment, the applied voltage to the light-emitting devices 25 is varied. In the foregoing Third Embodiment, the gain of the second amplifier 128 is varied. However, it is also possible to vary both the applied voltage to the light-emitting devices 25, and the gain of the second amplifier 128. With a wider variable range, the device life can be extended when changes occur over time.

## Claims

1. An information acquisition apparatus (1, 115, 126) comprising:
a first unit (2) including a photoreceiver (10) for receiving first reflected light reflected at an object (4), and outputting a signal corresponding to light intensity of the first reflected light; and
a second unit (3) separately provided from the first unit (2), the second unit (3) including a calibrator (36) having a stable reflectance and for outputting second reflected light to the photoreceiver (10), the second reflected light being used for comparing the light intensity of the first reflected light.

2. The information acquisition apparatus (1, 115, 126) according to claim 1, wherein the first unit (2) and the second unit include locating sections (5c, 41) with which the photoreceiver (10) and the calibrator (36) are installed face to face.

3. The information acquisition apparatus (1, 115, 126) according to claim 1 or 2, wherein the photoreceiver (10) includes:
a light-emitting device (25) for emitting light applied to the calibrator (36) or the object (4); and
a light-receiving device (33) for receiving the second reflected light or the first reflected light,
the light-emitting device and the light-receiving device having optical axes in the same direction.

4. The information acquisition apparatus (1, 115, 126) according to any of claims 1 to 3, wherein the calibrator (36) contains polytetrafluoroethylene.

5. The information acquisition apparatus (1, 115, 126) according to any of claims 1 to 4, wherein the first unit (2) includes:
a glucose level arithmetic section (73) for computing a glucose level using an output signal from the photoreceiver of which signal corresponds to light intensity of the first reflected light;
a determining section (74) for comparing the glucose level with a determination value to determine whether the object is in an abnormal state; and
a warning section (9) for giving a warning when the obj ect is in an abnormal state.

6. The information acquisition apparatus (1, 115, 126) according to claim 5,
wherein the first unit (2) includes a sending section (51) for sending information of the glucose level, and
wherein the second unit (3) includes a receiving section (78) for receiving information of the glucose level, and a storage section (77) for storing information of the glucose level.

7. The information acquisition apparatus (1, 115, 126) according to claim 6, wherein the second unit includes an analysis arithmetic section (92) for analyzing information of the glucose level.

8. The information acquisition apparatus (1, 115, 126) according to claim 7,
wherein the analysis arithmetic section (92) selects a countermeasure for the object (4), and
wherein the second unit (3) includes a display section (42) for displaying the countermeasure.
